(19) **Europäisches Patentamt / European Patent Office / Office européen des brevets**

(11) **EP 3 349 734 B1**

(12) **FASCICULE DE BREVET EUROPEEN**

(45) Date de publication et mention de la délivrance du brevet:
**28.12.2022 Bulletin 2022/52**

(21) Numéro de dépôt: **16774536.3**

(22) Date de dépôt: **13.09.2016**

(51) Classification Internationale des Brevets (IPC):
**A61K 9/20** *(2006.01)*  **A61K 31/155** *(2006.01)*
**A61K 31/192** *(2006.01)*  **A61K 31/196** *(2006.01)*
**A61K 31/282** *(2006.01)*  **A61K 31/337** *(2006.01)*
**A61K 31/366** *(2006.01)*  **A61K 31/40** *(2006.01)*
**A61K 31/428** *(2006.01)*  **A61K 31/4439** *(2006.01)*
**A61K 31/496** *(2006.01)*  **A61K 31/5415** *(2006.01)*

(52) Classification Coopérative des Brevets (CPC):
**A61K 9/205; A61K 31/155; A61K 31/192;**
**A61K 31/196; A61K 31/282; A61K 31/337;**
**A61K 31/366; A61K 31/4015; A61K 31/407;**
**A61K 31/4439; A61K 31/496; A61K 31/5415**

(86) Numéro de dépôt international:
**PCT/FR2016/052303**

(87) Numéro de publication internationale:
**WO 2017/046506 (23.03.2017 Gazette 2017/12)**

(54) **UTILISATION DE FRACTIONS SOLUBLES, INSOLUBLES D'UN POLYMÈRE DE CYCLODEXTRINE OU DE LEURS MÉLANGES COMME EXCIPIENT DANS UN COMPRIMÉ**

VERWENDUNG VON LÖSLICHEN UND UNLÖSLICHEN ANTEILEN EINES CYCLODEXTRIN-POLYMERS UND VON MISCHUNGEN DAVON ALS HILFSSTOFF IN EINER TABLETTE

USE OF SOLUBLE AND INSOLUBLE FRACTIONS OF A CYCLODEXTRIN POLYMER AND OF MIXTURES THEREOF AS AN EXCIPIENT IN A TABLET

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.09.2015 FR 1558575**

(43) Date de publication de la demande:
**25.07.2018 Bulletin 2018/30**

(73) Titulaires:
- **Université de Lille**
  **59800 Lille (FR)**
- **Centre National de la Recherche Scientifique - CNRS**
  **75016 Paris (FR)**
- **INSERM - Institut National de la Santé et de la Recherche Médicale**
  **75013 Paris (FR)**
- **Centre Hospitalier Régional et Universitaire de Lille (CHRU)**
  **59000 Lille (FR)**

(72) Inventeurs:
- **MARTEL, Bernard**
  **59850 Nieppe (FR)**
- **BLANCHEMAIN, Nicolas**
  **59660 Merville (FR)**
- **FLAMENT, Marie-Pierre**
  **59133 Phalempin (FR)**
- **WILLART, Jean-François**
  **59491 Villeneuve D'ascq (FR)**
- **TABARY, Nicolas**
  **59800 Lille (FR)**
- **GARCIA FERNANDEZ, Maria José**
  **59800 Lille (FR)**

(74) Mandataire: **Plasseraud IP**
**31, rue des Poissonceaux**
**CS 40009**
**59044 Lille Cedex (FR)**

(56) Documents cités:
WO-A1-00/47630    WO-A1-83/00809
US-A- 4 547 572    US-A1- 2013 012 613

- MARGUERITE J. KUTYLA ET AL: "Cyclodextrin-Crosslinked Poly(Acrylic Acid): Adhesion and Controlled Release of Diflunisal and Fluconazole from Solid Dosage Forms", AAPS PHARMSCITECH, vol. 14, no. 1, mars 2013 (2013-03), pages 301-311, XP055268198, DOI: 10.1208/s12249-012-9903-3
- DATABASE WPI Section Ch, Week 200748 Thomson Scientific, London, GB; Class A96, AN 2007-482799 XP002756996, NIE S; PAN W; ZHANG S: "Insoluble drug delivery system based on water-soluble cyclodextrin", -& CN 1 879 887 A ((UYSH-N) UNIV SHENYANG PHARM) 20 décembre 2006 (2006-12-20)
- MARIA ESTHER ZUGASTI ET AL: "Influence of soluble and insoluble cyclodextrin polymers on drug release from hydroxypropyl methylcellulose tablets", DRUG DEVELOPMENT AND INDUSTRIAL PHARMACY, vol. 35, no. 10, 8 octobre 2009 (2009-10-08), pages 1264-1270, XP055268182, US ISSN: 0363-9045, DOI: 10.1080/03639040902882306
- FENYVESI E ET AL: "Properties of cyclodextrin polymer as a tabletting aid", CHEMICAL AND PHARMACEUTICAL BULLETIN, PHARMACEUTICAL SOCIETY OF JAPAN, JP, vol. 32, no. 2, 1 février 1984 (1984-02-01), pages 665-669, XP008180021, ISSN: 0009-2363, DOI: 10.1248/CPB.32.665

- CELEBI N ET AL: "STUDIES ON DIRECT COMPRESSION OF SPIRONOLACTONE TABLETS USING CYCLODEXTRINE POLYMER", DIE PHARMAZIE, GOVI VERLAG PHARMAZEUTISCHER VERLAG GMBH, ESCHBORN, DE, vol. 49, no. 10, 1 octobre 1994 (1994-10-01), pages 748-750, XP000469848, ISSN: 0031-7144
- FENYVESI E: "Cyclodextrin polymers in the pharmaceutical industry", JOURNAL OF INCLUSION PHENOMENA, vol. 6, no. 5, octobre 1988 (1988-10), pages 537-545, XP002756997,
- MARIO JUG ET AL: "Influence of hydroxypropyl-[beta]-cyclodextrin complexation on piroxicam release from buccoadhesive tablets", EUROPEAN JOURNAL OF PHARMACEUTICAL SCIENCES, vol. 21, no. 2-3, 1 février 2004 (2004-02-01), pages 251-260, XP055268231, NL ISSN: 0928-0987, DOI: 10.1016/j.ejps.2003.10.029
- KUTYLA MARGUERITE J ET AL: "Cyclodextrin-crosslinked poly(acrylic acid): Synthesis, physicochemical characterization and controlled release of diflunisal and fluconazole from hydrogels", INTERNATIONAL JOURNAL OF PHARMACEUTICS, ELSEVIER, NL, vol. 444, no. 1, 14 January 2013 (2013-01-14), pages 175-184, XP028984484, ISSN: 0378-5173, DOI: 10.1016/J.IJPHARM.2013.01.005

**Description**

L'invention est définie par les revendications. Tout objet qui ne tombe pas sous l'étendue de la protection revendiquée est fourni à titre d'information uniquement. La présente invention plus spécifiquement concerne l'utilisation d'une fraction insoluble dans l'eau, d'une fraction soluble dans l'eau ou d'un mélange d'une fraction insoluble dans l'eau et d'une fraction soluble dans l'eau d'un polymère de cyclodextrine en tant qu'excipient dans un comprimé, comme revendiquée, une composition compressible constituée d'une fraction insoluble dans l'eau, d'une fraction soluble dans l'eau ou d'un mélange d'une fraction insoluble dans l'eau et d'une fraction soluble dans l'eau d'un polymère de cyclodextrine et d'au moins un principe actif, comme revendiquée, ainsi qu'un comprimé comprenant une telle composition compressible, comme revendiqué.

Dans la présente demande, les termes « soluble(s) » et « insoluble(s) » sont utilisés de façon interchangeable avec les termes « soluble(s) dans l'eau » et « insoluble(s) dans l'eau » respectivement.

## ETAT DE LA TECHNIQUE

Parmi les formes galéniques sous lesquelles les principes actifs sont administrés, le comprimé tient une place particulièrement importante. Ces comprimés sont réalisés par compression de compositions contenant, outre le principe actif, un ou plusieurs excipients.

Le choix du procédé de compression dépend principalement de la composition à comprimer. Les trois principaux procédés utilisés afin de former un comprimé sont :

- la compression directe qui consiste à comprimer de manière directe et sans étape intermédiaire une composition formée par le(s) excipient(s) et le(s) principe(s) actif(s) sous forme de poudres,
- la compression après une étape de granulation humide qui consiste à mouiller et agglomérer une composition formée par le(s) excipient(s) et le(s) principe(s) actif(s) avec une solution liante puis la sécher et la calibrer pour obtenir un grain qui sera comprimé,
- la compression après une étape de granulation sèche qui consiste à comprimer une composition formée par le(s) excipient(s) et le(s) principe(s) actif(s) une première fois avec un compacteur puis la broyer en grains qui pourront subir la compression finale.

La compression directe est, comme son nom l'indique, plus directe et plus rapide, mais reste très rarement utilisée car seule une très faible proportion de particules possède des propriétés de compressibilité adaptées à ce procédé.

En effet, afin de pouvoir comprimer une composition et d'obtenir des comprimés satisfaisants, une bonne compressibilité est requise. La composition doit ainsi préférentiellement avoir un écoulement libre, se densifier sous la contrainte et acquérir de la cohésion, rester sous forme compacte une fois la force de compression retirée et la résistance à la rupture du comprimé final doit être suffisamment élevée.

Un procédé d'agglomération, comme par granulation humide ou par granulation sèche, est ainsi généralement mis en œuvre au préalable avec la plupart des excipients pour obtenir de bonnes propriétés de compression.

Un excipient désigne toute substance sans activité thérapeutique entrant dans la composition du médicament ou utilisé pour sa fabrication. Dans un médicament sous forme de comprimé, un excipient peut être utilisé par exemple dans un ou plusieurs des objectifs suivants :

- permettre ou faciliter la préparation du comprimé,
- permettre ou faciliter l'emploi du comprimé,
- permettre la conservation du comprimé,
- assurer la stabilité chimique et l'état physique du principe actif au cours du temps,
- permettre la libération du principe actif à partir du comprimé,
- et contrôler la cinétique de libération du principe actif à partir du comprimé.

Parmi les excipients largement répandus et utilisés dans les comprimés pharmaceutiques figurent des polymères tels que les polysaccharides, les dérivés cellulosiques, les amidons et les dérivés acryliques.

Les cyclodextrines notamment sont connues en tant qu'excipient pour leurs propriétés de formation de complexe d'inclusion avec des molécules hydrophobes qui améliorent la biodisponibilité de ces molécules. Le document CN 1 879 887 A divulgue des comprimés comprenant un polymère de cyclodextrine soluble dans l'eau qui est réticulé par un éther bis-époxypropyle ou par de l'époxychloropropane (épichlorhydrine), et un principe actif insoluble dans l'eau. Les caractéristiques physicochimiques d'un excipient influencent sa fonctionnalité et permettent de garantir que la composition pharmaceutique présente les propriétés physiques et biopharmaceutiques requises. Dans le cas des comprimés, les industriels sont très demandeurs d'excipients qui, en plus de présenter une bonne aptitude à la compression, sont multifonctionnels. De tels excipients sont particulièrement utiles pour diminuer le nombre d'ingrédients dans le comprimé.

Par exemple, à l'heure actuelle, les comprimés à base d'ibuprofène (acide 2-(4-isobutylphényl)propanoïque) comme principe actif sont formés par un procédé de granulation humide après ajout de nombreux excipients comme de la silice afin d'éviter le collage de l'ibuprofène pendant la granulation, des agents désagrégeants, des agents liants et des agents lubrifiants.

Parmi les fonctionnalités recherchées, l'amélioration de la solubilité de principes actifs est un axe d'innovation important. En effet, les principes actifs étant très souvent insolubles en milieux aqueux ou sensibles aux conditions extérieures ou peu stables une fois délivrés dans l'organisme, la formulation d'excipients capables d'améliorer ces propriétés (solubilité, disponibilité, protection...) est un axe d'innovation important pour l'industrie pharmaceutique.

Les principes actifs purs sont souvent des composés obtenus sous une forme cristalline. Cependant, l'état cristallin est aussi celui qui présente la moins bonne solubilité et donc la moins bonne biodisponibilité. Ainsi, cette forme cristalline ne présente pas les propriétés thérapeutiques optimales. Augmenter la solubilité des principes actifs peu solubles apparaît donc clairement comme un des principaux challenges actuels du génie pharmaceutique.

L'une des principales solutions envisagées pour augmenter la solubilité et donc la biodisponibilité des principes actifs est de les formuler à l'état amorphe qui présente l'avantage d'être beaucoup plus soluble que l'état cristallin. L'état amorphe présente par contre l'inconvénient d'être intrinsèquement instable et de retourner vers l'état cristallin plus ou moins rapidement et de manière non contrôlée. La cinétique de recristallisation est alors essentiellement gouvernée par le degré de sous-refroidissement du principe actif par rapport à sa température de transition vitreuse (Tg).

Une solution possible pour stabiliser un principe actif de bas Tg à l'état amorphe est de réaliser une dispersion moléculaire de ce principe actif dans une matrice polymère (l'excipient principal du comprimé) de haut Tg. Cela fournit une solution solide vitreuse amorphe ayant un Tg intermédiaire situé entre celui du principe actif pur et celui du polymère excipient pur. Cette dispersion moléculaire amorphe a donc, à la fois, une meilleure solubilité que le principe actif cristallin pur et une meilleure stabilité physique que le principe actif amorphe pur.

Il est à noter que la stabilité physique d'une dispersion moléculaire peut être effective ou apparente. Elle est effective lorsque la concentration du principe actif dans le polymère (excipient) est inférieure à sa limite de solubilité. Dans ce cas, toute démixtion est impossible et la dispersion est parfaitement stable. La stabilité est par contre apparente lorsque la concentration du principe actif est supérieure à sa limite de solubilité et que le Tg du mélange reste élevé par rapport à la température de stockage. Dans ce cas, la démixtion du principe actif est inévitable, mais elle se produit sur des échelles de temps si longues qu'elle peut être négligée. Ces considérations mettent en évidence l'importance de disposer d'excipients pharmaceutiques de très haut Tg.

Par ailleurs, des excipients de complexation du principe actif, permettant de maîtriser la biodisponibilité et la dissolution dudit principe actif dans le milieu physiologique, sont également très recherchés à l'heure actuelle. Des excipients permettant d'obtenir une dissolution d'au moins 75% du principe actif en 45 min sont plus particulièrement souhaités afin de respecter les spécifications de la pharmacopée européenne pour les formes à libération conventionnelle.

Enfin, des excipients ayant des propriétés de désagrégation peuvent être utiles en ce qu'ils permettent une désagrégation plus rapide du comprimé.

Il existe aujourd'hui un besoin pour de nouveaux excipients multifonctionnels performants dans la solubilisation de principes actifs peu hydrophiles, présentant en particulier des propriétés mécaniques suffisantes pour former des comprimés, mais présentant également des capacités de solubilisation d'un principe actif et de complexation du principe actif de manière appropriée.

Par ailleurs, un autre but de l'invention consiste à fournir de nouveaux excipients présentant à la fois ces propriétés de compression, de dispersion, de solubilisation et de complexation du principe actif mais jouant également le rôle de liant et d'agent désagrégeant du comprimé.

En effet, à la connaissance des inventeurs, il n'existe actuellement pas un excipient multifonctionnel qui présenterait, de manière avantageuse, ces propriétés de compression, de dispersion de principe actif, de désagrégation du comprimé, de solubilisation et libération de principes actifs réunies.

## RESUME DE L'INVENTION

Il est du mérite des inventeurs d'avoir trouvé que la séparation d'un polymère de cyclodextrine en ses fractions insoluble et soluble dans l'eau donnait accès à des excipients multifonctionnels dont les propriétés, notamment de compression, de dispersion de principe actif, de solubilisation et de complexation de principes actifs, de désagrégation du comprimé peuvent être modulées en fonction des besoins grâce au mélange des deux fractions.

Ainsi, selon le but recherché, on peut utiliser la fraction insoluble seule, la fraction soluble seule ou un mélange quelconque des deux fractions.

Dans le cadre de la présente invention, il est important que le polymère de cyclodextrine soit utilisé sous forme

4

de fractions insoluble et/ou soluble dans l'eau, purifiées et isolées séparément, et non tel qu'obtenu à l'issue de la réaction de polymérisation, c'est-à-dire sous forme d'un mélange non défini contenant les fractions solubles et insolubles de polymère de cyclodextrine non séparées, des cyclodextrines monomères modifiées par l'agent de réticulation et éventuellement des produits de départ n'ayant pas réagi (la cyclodextrine et l'agent réticulant) ou d'autres impuretés telles que des traces de catalyseur utilisé pour promouvoir la réaction de polymérisation. Les fractions insoluble et soluble de polymères de cyclodextrine au sens de la présente invention sont obtenues à partir de polymères de cyclodextrine synthétisés selon les méthodes connues de l'homme du métier. Généralement, le polymère de cyclodextrine brut obtenu à l'issue de l'étape de polymérisation est mis en suspension dans de l'eau, puis les fractions solubles et insolubles sont séparées par filtration ; la fraction soluble est ensuite purifiée par exemple par dialyse ou ultrafiltration, puis atomisée ou lyophilisée, et la fraction insoluble est purifiée par lavages à l'eau distillée, séchée puis broyée. Les fractions insolubles et solubles purifiées obtenues sous forme de poudres sont ensuite utilisées soit séparément soit en mélange entre elles, le mélange de ces deux fractions permettant de contrôler la désagrégation du comprimé et la libération du principe actif en fonction des besoins en adaptant les proportions.

La présente invention concerne donc tout d'abord l'utilisation d'une fraction insoluble dans l'eau d'un polymère de cyclodextrine, d'une fraction soluble dans l'eau d'un polymère de cyclodextrine ou d'un mélange de fractions insoluble dans l'eau et soluble dans l'eau de polymères de cyclodextrine en tant qu'excipient unique pour un comprimé, telle que définie dans la revendication 1.

La présente invention concerne aussi l'utilisation d'un mélange de fractions insoluble dans l'eau et soluble dans l'eau de polymères de cyclodextrine en tant qu'excipient dans un comprimé, telle que définie dans la revendication 9. Au sens de la présente invention, le mélange de fractions insoluble et soluble est obtenu à partir de fractions insoluble et soluble préalablement séparées.

Un autre objet de l'invention se rapporte à une composition compressible constituée d'une fraction insoluble dans l'eau d'un polymère de cyclodextrine, d'une fraction soluble dans l'eau d'un polymère de cyclodextrine ou d'un mélange de fractions insoluble dans l'eau et soluble dans l'eau de polymères de cyclodextrine et d'au moins un principe actif, telle que définie dans la revendication 13.

Un autre objet de l'invention se rapporte à un comprimé comprenant une composition compressible telle que définie ci-dessus.

Enfin, la présente invention se rapporte à un procédé de fabrication d'un comprimé comprenant les étapes consistant à :

- préparer une composition compressible selon l'invention ;
- comprimer ladite composition compressible afin de former un comprimé.

## DESCRIPTION DETAILLEE DE L'INVENTION

Les cyclodextrines en tant que telles, c'est-à-dire les cyclodextrines sous forme de monomères, sont largement utilisées en tant qu'excipients. Les cyclodextrines sont des oligosaccharides cycliques composés de sous-unités glucopyranose liées en $\alpha$-(1,4). Les cyclodextrines naturelles ont six, sept ou huit unités (nommées respectivement $\alpha$-, $\beta$-, $\gamma$-cyclodextrine) et sont obtenues à partir de l'amidon. Les anhydroglucoses sont orientés dans le macrocycle de sorte que l'intérieur de la cavité est apolaire et hydrophobe, tandis que les hydroxyles primaires et secondaires sont présents sur la surface extérieure des cyclodextrines, assurant leur solubilité dans les milieux aqueux. Les cyclodextrines peuvent former ainsi des molécules cages capables de piéger des molécules organiques ou une partie de molécules organiques de faible polarité, pour former des complexes d'inclusion, avec une large variété de molécules organiques à caractère lipophile, en leur apportant une solubilité accrue dans l'eau, une protection contre l'hydrolyse, l'oxydation, une réduction de la volatilité, le masquage du goût etc.

Cependant, les cyclodextrines ne possèdent pas de capacités optimales de compression. Ainsi, les cyclodextrines sous forme de monomères forment notamment une poudre dont la faible fluidité empêche l'écoulement libre indispensable pour une bonne compression, et l'ajout d'excipients est nécessaire à l'obtention d'une cohésion suffisante. Par ailleurs, bien que les cyclodextrines possèdent un pouvoir de solubilisation, celui-ci reste limité pour certaines d'entre elles, notamment pour la $\beta$-cyclodextrine, à cause de sa faible solubilité dans l'eau (18,5 g/L à 20°C).

Les inventeurs ont découvert de manière surprenante et après de nombreuses recherches que, contrairement à une cyclodextrine sous forme de monomère ou même un polymère de cyclodextrine, les fractions insoluble et soluble utilisées séparément ou en mélange entre elles permettaient de réunir avantageusement toutes les propriétés souhaitées sans qu'un autre excipient ne soit nécessaire, en particulier en présentant de bonnes propriétés de compression via un écoulement libre, une bonne résistance à la compression ou encore une dureté de comprimé final élevée, un fort pouvoir de désagrégation du comprimé en présence d'eau, tout en présentant un fort potentiel de solubilisation et de vectorisation de principes actifs.

Cette utilisation avantageuse d'une fraction insoluble d'un polymère de cyclodextrine, d'une fraction soluble d'un polymère de cyclodextrine ou d'un mélange de fractions insolubles et solubles de polymères de cyclodextrine en tant qu'excipient pour un comprimé n'avait jamais été mise en évidence auparavant.

Ainsi, la demande IN 2010MU03419 A se rapporte à l'utilisation de polymères de cyclodextrine dans des formulations galéniques à libération prolongée de principe actif. Cependant, selon la description faite dans ce document, un mélange solide brut issu directement de l'étape de polymérisation non caractérisé à base de polymères de cyclodextrine insolubles est obtenu et utilisé sans aucune étape de purification en fin de réaction préalablement à la réalisation de la préparation galénique. Ainsi il n'est pas démontré que ce mélange non caractérisé et non purifié puisse conduire à l'obtention d'un comprimé dont les vitesses de désagrégation et de libération de l'actif sont contrôlables. De plus, un tel mélange solide brut, non purifié et non caractérisé, et contenant du catalyseur potentiellement utilisé issu directement du procédé de polymérisation ne saurait être homologué pour une utilisation en tant qu'excipient pharmaceutique.

Comme mis en évidence dans les exemples, les inventeurs ont réussi à obtenir, sans nécessiter d'autres excipients que la fraction insoluble d'un polymère de cyclodextrine, la fraction soluble d'un polymère de cyclodextrine ou un mélange de fractions insoluble et soluble de polymères de cyclodextrine (les fractions ayant été isolées par une étape de mise en suspension du mélange solide obtenu après polymérisation, une étape de filtration pour isoler la partie insoluble, une étape de dialyse ou ultrafiltration du filtrat (membrane 6-8000 Da) pour éliminer oligomères, cyclodextrine monomère ayant réagi avec l'agent réticulant dont la masse molaire est inférieure à 6000 Da, cyclodextrine et agent réticulant qui n'ont pas réagi et catalyseur, et enfin une étape d'atomisation du dialysat), des comprimés ayant typiquement un diamètre de 3 à 20 mm présentant une résistance à la rupture, comprise entre 10 et 120 N lors de compressions avec des forces de compression satisfaisantes n'excédant pas 2000 daN, tout en ayant des forces résiduelles, des forces à l'éjection et des indices de cohésion satisfaisants.

Par ailleurs, il a été mis en évidence de manière particulièrement intéressante que les fractions insolubles, les fractions solubles de polymères de cyclodextrine et les mélanges de ces fractions possèdent un fort potentiel de complexation de principes actifs. En particulier, il a été observé que la fraction soluble dans l'eau du polymère de cyclodextrine permettait une amélioration de la solubilité de principes actifs faiblement ou peu solubles dans l'eau en milieu aqueux. Ceci est particulièrement avantageux lors de l'utilisation de principes actifs, notamment pharmaceutiques, peu solubles, voire insolubles, comme par exemple l'ibuprofène (acide 2-[4-isobutylphényl]propanoïque), la simvastatine (2,2-diméthylbutanoate de (1S,3R,7S,8S,8aR)-8-{2-[(2R,4R)-4-hydroxy-6-oxotétrahydro-2H-pyran-2-yl]ethyl}-3,7-diméthyl-1,2,3,7,8,8a-hexahydro-1-naphthalényle) ou le piroxicam (1,1-dioxyde de 4-hydroxy-2-méthyl-N-(2-pyridinyl)-2H-1,2-benzothiazine-3-carboxamide).

Enfin, les inventeurs ont mis en évidence que l'utilisation de fractions solubles de polymères de cyclodextrine ou de mélanges de fractions soluble et insoluble tels qu'entendus dans la présente invention permettait d'obtenir une meilleure biodisponibilité du principe actif. En effet, la fraction soluble utilisée seule ou en mélange avec la fraction insoluble joue le rôle d'agent de complexation en encapsulant les principes actifs, même ceux de très faible solubilité, ce qui améliore sensiblement la solubilité du principe actif en milieu aqueux, dans une solution tampon ou dans le milieu physiologique. Par ailleurs, le polymère de cyclodextrine joue un rôle de vecteur de ce principe actif favorisant son transport vers les muqueuses du tractus gastro-intestinal où il est adsorbé avant de diffuser dans le circuit sanguin et atteindre les organes cibles.

Une fraction insoluble d'un polymère de cyclodextrine, une fraction soluble d'un polymère de cyclodextrine ou un mélange de fractions insoluble et soluble de polymères de cyclodextrine tels qu'entendus dans la présente invention peuvent donc jouer un rôle à la fois en tant qu'excipient pour la compression mais également en tant qu'excipient de solubilisation et/ou excipient de complexation. L'utilisation de cyclodextrines sous forme de monomère ou un polymère de cyclodextrine en tant que tel ne permettait pas l'obtention de telles propriétés avantageuses. Le fractionnement du polymère en ses fractions insoluble et soluble et éventuellement le mélange de ces deux fractions préalablement séparées et purifiées, aboutit à l'obtention d'un excipient pour comprimé présentant des propriétés de désintégration souhaitées et de contrôle de la vitesse de libération de principes actifs qui peuvent être adaptés en fonction des besoins en variant les proportions des deux fractions.

Par ailleurs, il a également été mis en évidence par les inventeurs que le mélange d'une fraction insoluble de polymère de cyclodextrine à une fraction soluble de polymère de cyclodextrine permettait d'obtenir de manière avantageuse des propriétés de désagrégation du comprimé grâce à un pouvoir gonflant très important en présence d'eau (de l'ordre de 500% par rapport au poids sec du polymère insoluble, c'est-à-dire qu'un gramme de fraction insoluble de polymère de cyclodextrine est capable d'absorber de l'ordre de cinq fois son poids en eau). Ainsi, les inventeurs ont découvert qu'il était possible d'obtenir un effet désagrégeant intéressant en plus des propriétés mécaniques avantageuses pour la compression, la solubilisation et la biodisponibilité obtenues avec la fraction soluble du polymère de cyclodextrine en utilisant également la fraction insoluble de polymère de cyclodextrine.

Selon un premier aspect, la présente invention concerne l'utilisation d'une fraction insoluble d'un polymère de cyclodextrine, d'une fraction soluble d'un polymère de cyclodextrine, ou d'un mélange de fractions insolubles et solubles de polymères de cyclodextrine en tant qu'excipient unique pour un comprimé, telle que définie dans la revendication 1.

Selon un deuxième aspect, la présente invention concerne l'utilisation d'un mélange de fractions insoluble dans l'eau et soluble dans l'eau de polymères de cyclodextrine en tant qu'excipient dans un comprimé, telle que définie dans la revendication 9.

Comme expliqué ci-dessus, les fractions insoluble et soluble utilisées séparément ou en mélange entre elles permettent, contrairement à une cyclodextrine sous forme de monomère ou même un polymère de cyclodextrine, de réunir avantageusement toutes les propriétés souhaitées sans qu'un autre excipient ne soit nécessaire, en particulier en présentant de bonnes propriétés de compression via un écoulement libre, une bonne résistance à la compression ou encore une dureté de comprimé final élevée, un fort pouvoir de désagrégation du comprimé en présence d'eau, tout en présentant un fort potentiel de solubilisation et de vectorisation de principes actifs. Ainsi, dans un aspect particulier, l'invention concerne l'utilisation d'une fraction insoluble d'un polymère de cyclodextrine, d'une fraction soluble d'un polymère de cyclodextrine, ou d'un mélange de fractions insolubles et solubles de polymères de cyclodextrine en tant qu'excipient unique pour un comprimé, comme revendiquée.

En particulier, la présente invention concerne l'utilisation d'une fraction insoluble d'un polymère de cyclodextrine, d'une fraction soluble d'un polymère de cyclodextrine, ou un mélange de fractions insolubles et solubles de polymères de cyclodextrine, comme revendiquée, en tant qu'excipient de compression, notamment de compression directe ou compression après une étape de granulation humide, excipient de solubilisation et/ou excipient de complexation pour un comprimé.

Selon un premier mode de réalisation, tel que défini dans les revendications, on utilise une fraction insoluble d'un polymère de cyclodextrine en tant qu'excipient pour un comprimé, comme par exemple en tant qu'excipient de compression, notamment de compression directe, excipient de solubilisation et/ou excipient de complexation pour un comprimé.

L'utilisation d'une fraction insoluble seule en tant qu'excipient pour un comprimé donne des comprimés avec un temps de désagrégation très court, tout en présentant une résistance à la rupture élevée. De plus, la fraction insoluble seule présente un écoulement libre ce qui la rend utilisable comme excipient de compression directe.

Une fraction insoluble d'un polymère de cyclodextrine en présence d'un excès d'eau présente en effet un taux de gonflement avantageux, de préférence supérieur à 100%, de préférence encore compris entre 100 et 1000%, ledit pourcentage étant exprimé par rapport au poids initial de la fraction insoluble de polymère de cyclodextrine sec. Ces capacités d'absorption d'eau permettent une désagrégation *in situ* dans le système digestif, dans un verre d'eau ou par voie orodispersible, permettant la libération rapide d'un principe actif à partir d'un comprimé.

Selon un deuxième mode de réalisation, tel que défini dans les revendications, on utilise une fraction soluble d'un polymère de cyclodextrine en tant qu'excipient pour un comprimé, comme par exemple en tant qu'excipient de compression, notamment de compression directe ou de compression après une étape de granulation humide, excipient de solubilisation et/ou excipient de complexation pour un comprimé.

L'utilisation d'une fraction soluble seule en tant qu'excipient pour un comprimé donne des comprimés avec un temps de désagrégation nettement supérieur à ceux obtenus avec la fraction insoluble seule mais toujours très inférieur à celui d'un comprimé préparé avec de la cyclodextrine (monomère) en tant qu'excipient de compression.

Selon une variante de ce mode de réalisation, la fraction soluble d'un polymère de cyclodextrine mise en œuvre en tant qu'excipient pour un comprimé est une fraction soluble granulée par voie humide. La granulation par voie humide présente l'avantage de conférer un écoulement libre à la fraction soluble ce qui la rend compressible, tout en maintenant le temps de désagrégation d'une fraction soluble non granulée.

La fraction soluble de polymère de cyclodextrine peut être granulée seule ou en mélange avec au moins un principe actif.

De manière générale, la granulation humide peut comprendre les étapes suivantes :

- mélange de la fraction soluble du polymère de cyclodextrine, d'un solvant et éventuellement au moins un principe actif afin de former une masse humide,
- granulation, notamment par passage forcé de la masse humide au travers d'une grille ou d'un tamis,
- séchage du grain obtenu à l'issue de l'étape de granulation, de préférence à une température comprise entre 40 et 80°C afin d'obtenir une poudre,
- éventuellement, tamisage de la poudre afin d'obtenir une poudre de granulométrie comprise entre 250 et 1000 $\mu$m, de préférence entre 400 et 700 $\mu$m, de préférence encore inférieure à 630 $\mu$m.

N'importe quel solvant approprié peut être utilisé pour la granulation. De préférence, le solvant est choisi parmi les solvants aqueux, alcooliques et hydro-alcooliques. De préférence encore, le solvant est de l'eau. Ainsi, de manière avantageuse, le seul excipient utilisé lors de cette étape de granulation humide est la fraction soluble d'un polymère de cyclodextrine. La fraction soluble de polymère de cyclodextrine a en effet toutes les propriétés nécessaires pour la mise en œuvre d'une granulation humide et aucun autre excipient, par exemple un agent liant, n'est requis.

La composition compressible obtenue à l'issue de la granulation humide possède des caractéristiques avantageuses pour la mise en œuvre de la compression.

Typiquement, la composition compressible a une densité après tassement comprise entre 0,5 et 0,8 g/mL, et de préférence 0,625 g/mL.

Par ailleurs, la composition compressible a typiquement un indice de compressibilité (Indice de Carr, Ic) compris entre 1 et 20. L'indice de compressibilité est déterminé par la méthode Carr en utilisant par exemple un Engelmann powder tester (Pharma Test Instruments, Inde).

Selon un troisième mode de réalisation, tel que défini dans les revendications, on utilise un mélange d'une fraction insoluble et d'une fraction soluble d'un polymère de cyclodextrine en tant qu'excipient pour un comprimé, comme par exemple en tant qu'excipient de compression, notamment de compression directe, excipient de solubilisation et/ou excipient de complexation pour un comprimé.

Ce mode de réalisation permet d'ajuster les propriétés de l'excipient en fonction des besoins. Typiquement, plus la fraction insoluble de polymère de cyclodextrine est en quantité importante, plus la désagrégation du comprimé dans un milieu aqueux sera rapide. Inversement, la fraction soluble de polymère de cyclodextrine est très efficace dans la complexation des principes actifs et est avantageuse vis-à-vis de l'effet de libération d'un principe actif dans un milieu aqueux et ainsi sa vectorisation dans les fluides physiologiques est importante. Un mélange de fractions insoluble et soluble d'un polymère de cyclodextrine avec un faible taux de fraction soluble inférieur à 20% en poids (sec/sec) permet de conserver un écoulement libre et un temps de désintégration rapide tandis que des mélanges présentant un taux de fraction soluble supérieur ou égal à 20% en poids (sec/sec), ne présentent plus d'écoulement libre et un temps de désagrégation de l'ordre de celui obtenu pour un comprimé préparé avec la fraction soluble seule d'un polymère de cyclodextrine. Ainsi, le ratio fraction insoluble de polymère de cyclodextrine / fraction soluble de polymère de cyclodextrine peut être fixé selon l'effet souhaité.

Si une désagrégation rapide est souhaitée, le ratio fraction insoluble de polymère de cyclodextrine / fraction soluble de polymère de cyclodextrine est compris de préférence entre 100:0 et 0:100, de préférence encore entre 100:0 et 80:20.

Si un effet de complexation/resolubilisation de principes actifs est souhaité, le ratio fraction insoluble de polymère de cyclodextrine / fraction soluble de polymère de cyclodextrine est compris de préférence entre 80:20 et 0:100 et, de préférence encore entre 25:75 et 0:100.

Selon un troisième aspect, la présente invention concerne une composition compressible constituée d'une fraction insoluble d'un polymère de cyclodextrine, d'une fraction soluble d'un polymère de cyclodextrine, ou un mélange de fractions insolubles et solubles de polymères de cyclodextrine et d'au moins un principe actif, telle que définie dans la revendication 13. Sans vouloir être lié par une quelconque théorie, les inventeurs pensent qu'il se forme un complexe entre le principe actif et la fraction insoluble et/ou la fraction soluble lors de leur mélange.

Selon un premier mode de réalisation, tel que défini dans les revendications, la composition compressible est constituée d'une fraction insoluble d'un polymère de cyclodextrine et d'au moins un principe actif.

Selon un deuxième mode de réalisation, tel que défini dans les revendications, la composition compressible est constituée d'une fraction soluble d'un polymère de cyclodextrine et d'au moins un principe actif. Selon une variante de ce mode de réalisation, la fraction soluble d'un polymère de cyclodextrine a été mélangée avec au moins un principe actif puis granulée par voie humide.

Un écoulement libre est obtenu comparativement à la fraction soluble de polymère de cyclodextrine n'ayant pas subi l'étape de granulation humide; le temps de désagrégation est de l'ordre de celui obtenu pour un comprimé préparé avec la fraction soluble du polymère de cyclodextrine n'ayant pas subi l'étape de granulation humide.

Selon un troisième mode de réalisation, tel que défini dans les revendications, la composition compressible est constituée d'un mélange d'une fraction insoluble et d'une fraction soluble d'un polymère de cyclodextrine. Selon une variante de ce mode de réalisation, la fraction soluble d'un polymère de cyclodextrine a été mélangée avec au moins un principe actif puis granulée par voie humide. Selon une autre variante de ce mode de réalisation particulier, la fraction insoluble est mélangée avec au moins un principe actif. Les avantages de ces différents modes de réalisations sont détaillés ci-dessus par rapport à l'utilisation de fractions insoluble et soluble, séparément ou en mélanges entre elles comme excipient pour comprimés. Dans la présente invention, on désigne par « composition compressible » une composition sous forme de poudre utilisable, per se, pour la fabrication de comprimés par compression. Aucun excipient supplémentaire n'est nécessaire afin de permettre la compression. Une telle composition permet la fabrication de comprimés de paramètres physico-mécaniques appropriés et d'apparence satisfaisante.

On entend par «polymère de cyclodextrine », au sens de l'invention, un polymère de cyclodextrine réticulé par un acide poly(carboxylique) obtenu par condensation des groupes hydroxyles de la cyclodextrine avec les fonctions carboxyliques de l'acide poly(carboxylique), ou un mélange de polymères de cyclodextrine réticulés par un acide poly(carboxylique), ou encore un copolymère d'au moins deux cyclodextrines réticulé par un acide poly(carboxylique). Dans la présente invention, l'acide poly(carboxylique) est l'acide citrique, et un tel polymère de cyclodextrine se présente sous forme de poudre.

Ainsi, le terme « fraction soluble de polymère de cyclodextrine » désigne la fraction soluble d'un polymère de cyclodextrine ou un mélange de fractions solubles de polymères de cyclodextrine ou encore la fraction soluble d'un copolymère de cyclodextrines. À l'inverse, le terme « fraction insoluble de polymère de cyclodextrine » désigne la fraction insoluble d'un polymère de cyclodextrine ou un mélange de fractions insolubles de polymères de cyclodextrine ou la fraction insoluble d'un copolymère de cyclodextrines. L'expression « mélange d'une fraction insoluble et d'une fraction soluble d'un polymère de cyclodextrine » quant à elle désigne un mélange préparé à partir de fractions soluble(s) et insoluble(s) de cyclodextrine préalablement séparés et éventuellement purifiés. En d'autres termes pour préparer un « mélange d'une fraction insoluble et d'une fraction soluble d'un polymère de cyclodextrine » au sens de l'invention, le polymère de cyclodextrine est d'abord séparé en une ou plusieurs fractions solubles et une ou plusieurs fractions insolubles, puis ces fractions insoluble(s) et solubles(s) sont remélangées dans les proportions souhaitées. On entend par « cyclodextrine » une cyclodextrine sous forme de monomère, notamment l'a-cyclodextrine, la β-cyclodextrine ou l'γ-cyclodextrine, ou un dérivé de cyclodextrine, notamment les dérivés hydroxypropyl, méthyl, sulfobutylether ou carboxyméthyl de l'α-cyclodextrine, la β-cyclodextrine ou l'γ-cyclodextrine, ou encore le mélange de deux ou de plusieurs de ces cyclodextrines. De préférence, une cyclodextrine est choisie parmi une α-cyclodextrine, une β-cyclodextrine, une γ-cyclodextrine, un dérivé hydroxypropyl de l'α-cyclodextrine, un dérivé hydroxypropyl de la β-cyclodextrine, un dérivé hydroxypropyl de l'γ-cyclodextrine, un dérivé méthyl de l'α-cyclodextrine, un dérivé méthyl de la β-cyclodextrine et/ou un dérivé méthyl de l'γ-cyclodextrine. De préférence encore, une cyclodextrine est choisie parmi une α-cyclodextrine, une β-cyclodextrine, une γ-cyclodextrine, un dérivé hydroxypropyl de la β-cyclodextrine, un dérivé hydroxypropyl de l'γ-cyclodextrine et/ou un dérivé méthyl de la β-cyclodextrine. De préférence encore, un dérivé hydroxypropyl de la β-cyclodextrine et/ou un dérivé méthyl de la β-cyclodextrine.

L'acide poly(carboxylique) au sens de la présente invention est l'acide citrique.

Toute méthode connue de l'homme du métier permettant d'arriver à un polymère de cyclodextrine tel qu'entendu dans la présente invention peut ainsi être mise en œuvre. On peut par exemple se référer à la demande WO00/47630 ou encore à la publication Martel et al. (B. Martel, D. Ruffin, M. Morcellet, M. Weltrowski, Y. Lekchiri, Water soluble polymers and gels from polycondensation between cyclodextrins and polycarboxylic acids : study of the preparation parameters, J. Appl. Polym. Sci., 2005, 97, 433-442) qui décrivent un procédé de préparation de polymères de cyclodextrine. Les fractions solubles et insolubles mises en œuvre dans la présente invention sont ensuite obtenues par mise en suspension du mélange solide obtenu à l'issue de la réaction de polymérisation dans de l'eau, séparation des fractions soluble et insoluble par filtration, puis purification par exemple par dialyse ou ultrafiltration et ensuite atomisation ou lyophilisation, et lavage de la fraction insoluble à l'eau, suivi de son séchage.

Typiquement, la préparation de fractions insoluble et soluble de polymères de cyclodextrine comprend les étapes suivantes :

- préparation d'un mélange à l'état solide de cyclodextrine, d'un acide poly(carboxylique) et, éventuellement, d'un catalyseur ;
- chauffage du mélange solide, à une température comprise entre 100°C et 200°C, pendant une durée comprise entre 1 min et 100 min, de préférence, comprise entre 30 et 90 min ;
- mise en suspension du mélange solide dans l'eau ;
- séparation des fractions soluble et insoluble ;
- concentration de la fraction soluble, purification par exemple par dialyse ou ultrafiltration, puis atomisation ou lyophilisation ;
- lavage de la fraction insoluble à l'eau puis séchage.

Pour la première étape de préparation du mélange à l'état solide de cyclodextrine et d'un acide poly(carboxylique) et, éventuellement, d'un catalyseur, il est avantageux de préparer une solution aqueuse de cyclodextrine, d'un acide poly(carboxylique) et, éventuellement, d'un catalyseur, puis d'évaporer l'eau de la solution aqueuse en chauffant à une température comprise entre 50°C et 100°C. Cette étape de chauffage peut être effectuée sous pression réduite ou sous pression atmosphérique, de préférence sous pression réduite. De préférence encore, cette étape de chauffage est effectuée à une température comprise entre 50°C et 70°C sous pression réduite de 7,7 kPa (58 mmHg). Selon l'invention, l'acide poly(carboxylique) est l'acide citrique.

Le catalyseur utilisé est de préférence choisi parmi les dihydrogénophosphates, les hydrogénophosphates, les phosphates, les hypophosphites, les phosphites de métaux alcalins et d'ammonium, les sels de métaux alcalins des acides polyphosphoriques, les carbonates, les bicarbonates, les acétates, les borates, les hydroxydes de métaux alcalins, les amines aliphatiques et l'ammoniaque, et de préférence, parmi l'hydrogénophosphate de sodium, le dihydrogénophosphate de sodium et l'hypophosphite de sodium ainsi que l'hypophosphite d'ammonium.

Pour la seconde étape de chauffage du mélange solide, en général, un chauffage à température élevée, par exemple comprise entre 160°C et 200°C, pendant une durée supérieure à 30 min permettra d'obtenir majoritairement une fraction insoluble de polymère de cyclodextrine (rendement massique = 70%) et peu de fraction soluble de polymère, tandis qu'un chauffage à température moins élevée, par exemple comprise entre 120 et 140°C, pendant une durée comprise entre 20 et 60 min permettra d'obtenir une plus grande proportion de fraction soluble de polymère de cyclodextrine (rendement massique maximal = 40%). Cette étape de chauffage peut être effectuée sous pression réduite ou sous pression atmosphérique, de préférence sous pression réduite.

Après l'étape de chauffage, on peut récupérer la fraction soluble et/ou la fraction insoluble du polymère de cyclodextrine. Typiquement, le produit obtenu après chauffage est mis en suspension dans l'eau puis filtré, afin de séparer la fraction insoluble et la fraction soluble.

La fraction insoluble de polymère de cyclodextrine peut ensuite être séchée, par exemple à une température comprise entre 20°C et 70°C, afin d'être obtenue sous forme de poudre.

La fraction soluble de polymère de cyclodextrine peut subir plusieurs étapes de transformation afin de donner une poudre. De manière avantageuse, les étapes suivantes sont effectuées sur la fraction soluble de polymère de cyclodextrine obtenue après séparation:

- une étape de concentration,
- une étape de dialyse ou d'ultrafiltration, avec de préférence un seuil de coupure moléculaire compris entre 6 kDa et 50 kDa, de préférence encore un seuil de coupure moléculaire compris entre 6 kDa et 8 kDa,
- une étape d'atomisation ou de lyophilisation.

L'étape d'atomisation ou de lyophilisation permet ainsi d'obtenir la fraction soluble de polymère de cyclodextrine sous forme de poudre.

Dans un mode de réalisation préféré, la fraction soluble de polymère de cyclodextrine est atomisée. Les inventeurs ont mis en évidence que, contrairement à une poudre obtenue selon le procédé décrit dans la demande WO00/47630 comprenant une étape de lyophilisation, la poudre de fraction soluble de polymère de cyclodextrine selon l'invention obtenue par atomisation, présente intrinsèquement la propriété de compressibilité requise pour l'obtention de comprimé par compression directe. Cette propriété peut être optimisée par l'utilisation d'une buse d'atomisation de diamètre suffisamment élevé pour produire des particules de diamètre supérieur améliorant ainsi la fluidité. La fraction soluble de polymère de cyclodextrine lyophilisée quant à elle, ne présente pas de bonnes propriétés d'écoulement et présente un volume apparent très important du fait de sa très faible densité ce qui gêne considérablement la compression.

La fraction soluble de polymère de cyclodextrine tel qu'entendu dans la présente invention a une masse molaire moyenne en poids comprise entre 10 000 et 200 000 g.mol$^{-1}$. La masse molaire moyenne en poids est mesurée par chromatographie d'exclusion stérique.

La teneur en cyclodextrine de la fraction soluble du polymère de cyclodextrine selon la présente invention peut être déterminée par résonnance magnétique nucléaire (RMN), notamment par conversion de l'aire du signal des groupes méthylènes ($CH_2$) de l'agent réticulant (acide citrique) dont le déplacement chimique est d'environ 2,8 ppm (partie par million), et celle du proton H1 anomérique des unités glucoses de la cyclodextrine dont le déplacement chimique est d'environ 5 ppm. Ainsi la teneur en cyclodextrine varie entre 40 et 70% en poids, selon le taux de réticulation du polymère de cyclodextrine, qui dépend de la proportion d'acide citrique et de cyclodextrine introduite dans le réacteur, et aussi de la nature de la cyclodextrine ou du dérivé de cyclodextrine mis en jeu dans la synthèse, et enfin des paramètres de durée et de température appliqués lors du traitement thermique du mélange solide de cyclodextrine et d'acide citrique.

Selon un mode de réalisation particulier, le polymère de cyclodextrine selon la présente invention peut être préparé à partir d'une seule cyclodextrine choisie parmi l'a-cyclodextrine, la β-cyclodextrine, la γ-cyclodextrine et de leurs dérivés.

Selon un autre mode de réalisation particulier, le polymère de cyclodextrine selon la présente invention peut être préparé à partir de plusieurs cyclodextrines choisies parmi l'a-cyclodextrine, la β-cyclodextrine, la γ-cyclodextrine et leurs dérivés, tout simplement en introduisant un mélange de ces cyclodextrines dans le réacteur de polymérisation. Ainsi on peut obtenir par exemple un polymère contenant à la fois α-, β-, γ-cyclodextrine. Ceci apporte l'avantage d'une polyvalence du polymère, puisque celui-ci présentera un compromis optimum dans la solubilisation de principes actifs de tailles variables. Ainsi, un tel polymère pourra être utilisé comme excipient unique dans la préparation de divers comprimés dont les principes actifs seraient préférentiellement complexés par l'une ou l'autre des différentes cyclodextrines.

Comme décrit ci-dessus, la composition compressible selon l'invention est constituée d'une fraction insoluble d'un polymère de cyclodextrine, d'une fraction soluble d'un polymère de cyclodextrine, ou d'un mélange de fractions insolubles et solubles de polymères de cyclodextrine et d'au moins un principe actif, telle que définie dans la revendication 13.

Typiquement, la fraction soluble de polymère de cyclodextrine représente au moins 10% de ladite composition compressible, ledit pourcentage étant exprimé en poids sec par rapport au poids sec total de la composition compressible.

On entend par « principe actif » une molécule autre qu'un excipient qui est destinée, notamment à exercer une action pharmacologique et qui présente des propriétés thérapeutiques, préventives ou de diagnostic.

Selon l'invention, ledit au moins un principe actif est un principe actif insoluble choisi parmi l'ibuprofène, la simvastatine, le piroxicam, l'itraconazole, le diclofénac de sodium, la mitomycine, l'oméprazole, l'ethoxzolamide, la chlorhexidine, la ciprofloxacine, le cisplatine et/ou le paclitaxel.

En effet, les inventeurs ont mis en évidence que l'utilisation d'une fraction insoluble d'un polymère de cyclodextrine, d'une fraction soluble d'un polymère de cyclodextrine, ou d'un mélange de fractions insolubles et solubles de polymères de cyclodextrine, comme revendiquée, permettait la solubilisation de principe actif, en particulier de principe actif peu soluble voire insoluble. La solubilité du principe actif en combinaison avec une fraction soluble de polymère de cyclodextrine tel qu'entendu dans la présente invention est significativement améliorée en comparaison à la solubilité du même principe actif seul ou même en combinaison avec une cyclodextrine sous forme de monomère.

Ainsi, les fractions insoluble ou soluble d'un polymère de cyclodextrine, ou un mélange quelconque de ces fractions selon l'invention peuvent également être utilisés comme excipient de solubilisation dudit au moins un principe actif de la composition compressible selon l'invention.

Par ailleurs, les inventeurs ont mis en évidence que l'utilisation d'une fraction insoluble d'un polymère de cyclodextrine, d'une fraction soluble d'un polymère de cyclodextrine, ou d'un mélange de fractions insolubles et solubles de polymères de cyclodextrine, comme revendiquée, permettait d'obtenir, via la complexation du principe actif, une meilleure solubilisation dudit principe actif dans un milieu aqueux, une solution tampon ou dans un fluide physiologique. La dissolution du principe actif en combinaison avec une fraction insoluble d'un polymère de cyclodextrine, une fraction soluble d'un polymère de cyclodextrine ou un mélange de fractions insolubles et solubles de polymères de cyclodextrine soluble tel qu'entendu dans la présente invention est en effet significativement améliorée en comparaison à la dissolution du même principe actif seul ou même en combinaison avec une cyclodextrine sous forme de monomère. Il est possible d'obtenir une dissolution dans un milieu aqueux, une solution tampon ou un fluide physiologique du principe actif en combinaison avec une fraction soluble de polymère de cyclodextrine supérieure à 75% après 45 minutes, ce qui la rend conforme aux spécifications de la pharmacopée européenne (2.9.3. essai de dissolution des formes solides).

Ainsi, les fractions insoluble ou soluble d'un polymère de cyclodextrine, ou un mélange quelconque de ces fractions selon l'invention peuvent également être utilisés comme excipient de complexation dudit au moins un principe actif de la composition compressible selon l'invention.

La composition compressible constituée d'une fraction insoluble d'un polymère de cyclodextrine, d'une fraction soluble d'un polymère de cyclodextrine, ou d'un mélange de fractions insolubles et solubles de polymères de cyclodextrine et d'au moins un principe actif, comme revendiquée, peut être préparée selon toute méthode connue de l'homme du métier. En particulier, l'homme du métier utilisera une méthode de préparation permettant la formation d'un complexe entre la fraction insoluble ou soluble d'un polymère de cyclodextrine ou d'un mélange quelconque de ces deux fractions et ledit au moins un principe actif. Un tel complexe peut être obtenu par toute méthode connue de l'homme du métier, par exemple par granulation humide.

Dans ce cas, de manière particulièrement avantageuse, la composition compressible selon l'invention est sous forme de poudre de granulométrie comprise de préférence entre 250 et 1000 $\mu$m, de préférence entre 400 et 700 $\mu$m, de préférence encore inférieure à 630 $\mu$m. Une telle granulométrie de la composition compressible est avantageuse pour la mise en œuvre de la compression. La granulométrie souhaitée peut être obtenue par toute méthode connue de l'homme du métier, par exemple par granulation humide. Pour les besoins de la présente invention, la granulométrie est déterminée par tamisage.

Selon un quatrième aspect, la présente invention concerne un comprimé comprenant une composition compressible tel que définie ci-dessus.

Selon un mode de réalisation particulier, le comprimé selon l'invention est uniquement constitué de la composition compressible. Ladite composition compressible est en effet apte à être comprimée telle quelle, en particulier grâce aux propriétés avantageuses des fractions insoluble et soluble de polymère de cyclodextrine mis en œuvre séparément ou dans un mélange quelconque entre elles. Ce mode de réalisation est particulièrement

avantageux lorsqu'on utilise une fraction insoluble seule ou une fraction soluble granulée par voie humide seule ou un mélange d'une fraction insoluble et d'une fraction soluble ayant un taux de fraction soluble au plus égal à 10% en poids (sec/sec).

Selon un autre mode de réalisation particulier, le comprimé peut comprendre au moins un agent de compression, au moins un agent désagrégeant, au moins un agent liant, au moins un agent lubrifiant et/ou au moins un additif.

Selon ce mode de réalisation particulier, le comprimé comprend moins de 90%, de préférence moins de 80%, de préférence moins de 70%, de préférence moins de 60%, de préférence moins de 50%, de préférence encore moins de 40%, de préférence encore moins de 30%, de préférence encore moins de 20%, de préférence encore moins de 10%, de préférence encore moins de 5%, de préférence encore moins de 2%, de préférence encore moins de 1%, d'excipient autre que ladite fraction soluble de polymère de cyclodextrine.

Par exemple, le comprimé peut comprendre au moins un agent lubrifiant, par exemple du stéarate de magnésium, de préférence en une quantité inférieure à 2%, de préférence encore inférieure à 1%, de préférence d'environ 0,5%, ledit pourcentage étant exprimé en poids sec par rapport au poids sec total du comprimé.

Une fraction insoluble de polymère de cyclodextrine en présence d'eau a ainsi un taux de gonflement avantageux, de préférence supérieur à 100%, de préférence encore compris entre 100 et 1000%, ledit pourcentage étant exprimé par rapport au gain de poids après absorption d'eau de la fraction insoluble de polymère de cyclodextrine sèche. Ces capacités d'absorption d'eau provoquent une désagrégation rapide du comprimé mis en présence d'eau, de solution tampon ou de fluides physiologiques, permettant la libération rapide d'un ou plusieurs principes actifs.

Un objet de la présente invention concerne enfin un procédé de fabrication d'un comprimé comprenant les étapes consistant à :

- préparer au moins une composition compressible selon l'invention,
- comprimer ladite au moins une composition compressible afin de former un comprimé.

Selon un mode de réalisation particulier, ladite étape consistant à préparer une composition compressible est effectuée par granulation humide d'une fraction soluble d'un polymère de cyclodextrine et d'au moins un principe actif, notamment comme expliqué précédemment.

L'étape consistant à comprimer ladite au moins une composition compressible peut être effectuée selon toute méthode connue de l'homme du métier. On peut par exemple se référer à la méthode de compression mentionnée dans les exemples.

Un comprimé selon l'invention peut ainsi être formé à partir de la composition compressible, avec ou sans excipient supplémentaire. Ainsi, une fraction soluble, une fraction insoluble ou un mélange de fractions soluble et insoluble de polymère de cyclodextrine, selon l'invention, peut être utilisé comme excipient de compression dans un comprimé. Les inventeurs ont en effet mis en évidence que, contrairement à une cyclodextrine sous forme de monomère, l'utilisation d'une fraction soluble, d'une fraction insoluble ou d'un mélange de fractions soluble et insoluble de polymère de cyclodextrine, comme revendiquée, permettait de réunir avantageusement toutes les propriétés souhaitées sans qu'un autre excipient soit nécessaire, en particulier en présentant de bonnes propriétés de compression via un indice de compressibilité, une dureté et une résistance à la rupture de comprimé final élevés, et de bonnes propriétés de désagrégation.

Avantageusement, un comprimé selon l'invention a un poids compris entre 25 mg et 1500 mg et un diamètre compris entre 3 mm et 20 mm.

Un tel comprimé a de préférence une résistance à la rupture comprise entre 10 N et 120 N.

Avantageusement, un comprimé selon l'invention a une durée de désintégration comprise entre moins de 15 secondes et 15 minutes.

Ainsi, de manière avantageuse, un comprimé selon l'invention a un diamètre de 5 mm, un poids de 100 mg, une résistance à la rupture comprise entre 10 N et 120 N. L'invention est décrite plus en détail ci-après, à l'aide des figures et exemples suivants qui ne sont nullement limitatifs mais sont donnés à titre d'exemple uniquement.

**FIGURES**

**Figure 1** : Spectre RMN $^1$H de la fraction soluble du polyCTR-βCD.

**Figure 2** : Hygroscopicité de la βCD.

**Figure 3** : Hygroscopicité de la fraction soluble du PolyCTR-βCD.

**Figure 4** : Hygroscopicité de la fraction insoluble du PolyCTR-βCD.

**Figure 5** : Représentation de la distribution granulométrique de poudre de fraction soluble de PolyCTR-βCD obtenue par atomisation.

**Figure 6** : Clichés pris au microscope optique de poudre de poly-βCD atomisé dispersé dans de l'huile de paraffine avec un grossissement de 20x (a) et 50x (b) et de poudre de poly-βCD lyophilisé dispersé dans de l'huile de paraffine avec un grossissement de 20x (c).

**Figure 7 :** Diagramme de solubilité de l'ibuprofène avec la βCD, γCD et les fractions solubles de leurs polymères: PolyCTR-βCD, PolyCTR-γCD et PolyCTR-βCD/γCD.

**Figure 8 :** Diagramme de solubilité du piroxicam avec la βCD, yCD et les fractions solubles de leurs polymères: PolyCTR-βCD, PolyCTR-yCD et PolyCTR-pCD/yCD.

**Figure 9 :** Grandes étapes de la granulation humide. 1) Ajout d'un liant ; 2) Formation d'une liaison liquide ; 3) Transformation en une liaison solide par évaporation ; 4) Obtention d'une particule ronde.

**Figure 10 :** Image des grains CD (ou polyCD) / principe actif obtenu par granulation humide.

**Figure 11 :** Diffractogrammes de la poly-βCD, la poly-MebCD (poly-crysmeb), la βCD et la MebCD (crysmeb).

**Figure 12 :** Thermogrammes de solutions vitreuses $(poly-\beta CD)_{1-x}(mannitol)_x$ enregistrés au chauffage (5°C/min).

**Figure 13 :** Diffractogrammes enregistrés à 20°C et thermogrammes enregistrés au chauffage (5°C/min) de la simvastatine, d'un mélange simvastatine/βCD et d'un mélange simvastatine/poly-βCD. Les mélanges ont été obtenus par granulation humide.

**Figure 14 :** Thermogrammes enregistrés au chauffage (5°C/min) du piroxicam, d'un mélange piroxicam/poly-βCD et d'un mélange piroxicam/βCD ayant tous les trois subi un traitement préalable de fusion trempe à 210°C.

**Figure 15 :** Comprimés de fraction soluble de polymère obtenus par compression.

**Figure 16 :** Dureté de la fraction soluble du polymère seule en fonction de la force de compression mesurée au niveau du poinçon supérieur.

**Figure 17** : Courbes de dissolution de l'ibuprofène seul ou granulé avec la βCD ou la fraction soluble de PolyCTR-βCD (50 mL/min, 37°C, 500 mL HCl pH = 1).

**Figure 18** : Courbes de dissolution de l'ibuprofène seul ou granulé avec la HPβCD ou la fraction soluble de PolyCTR-HPβCD (50 mL/min, 37°C, 500 mL HCl pH = 1).

**Figure 19** : Courbes de dissolution de l'ibuprofène seul ou granulé avec la MeβCD ou la fraction soluble de PolyCTR-MeβCD (50 mL/min, 37°C, 500 mL HCl pH = 1).

**Figure 20 :** Courbes de dissolution du piroxicam seul ou granulé avec la βCD ou la fraction soluble de PolyCTR-βCD (50 mL/min, 37°C, 500 mL HCl pH = 4) en comparaison avec le comprimé commercial.

**Figure 21** : Courbes de dissolution du piroxicam seul ou granulé avec la HPβCD ou la fraction soluble de PolyCTR-HPβCD (50 mL/min, 37°C, 500 mL HCl pH = 4) en comparaison avec le comprimé commercial.

## EXEMPLES

### Exemple 1 : Synthèse de fractions solubles et insolubles de polymères de cyclodextrine

Les cyclodextrines suivantes ont été utilisées:

- β-cyclodextrine (βCD) (Roquette, Lestrem, France)
- γ-cyclodextrine (γCD) (Wacker, Bürghausen, Germany),
- hydroxypropyl-β-cyclodextrine (HPβCD1) (Kleptose®HP, MS = 0.85, Roquette, Lestrem, France)
- hydroxypropyl-β-cyclodextrine (HPβCD2), (Kleptose®HPB, MS = 0.62, Roquette, Lestrem, France)
- méthyl-β-cyclodextrine (MeβCD) (Crysmeb®, DS = 0.50, Roquette, Lestrem, France)
- hydroxypropyl-γ-cyclodextrine (HPγCD) (Wacker, Bürghausen, Germany).
- Maltodextrine (MD)

Les polymères de MD, βCD, γCD, HPβCD1, HPβCD2, MeβCD et HPγCD, appelés respectivement PolyCTR-MD, PolyCTR-βCD, PolyCTR-γCD, PolyCTR-HPβCD1, PolyCTR-HPβCD2, PolyCTR-MeβCD et PolyCTR-HPγCD ont été synthétisés en utilisant l'acide citrique (CTR) comme agent réticulant dans un réacteur semi-industriel (20 L) selon une méthode adaptée qui permettait la production du polymère à l'échelle laboratoire (demande WO00/47630). Une solution aqueuse (1000 mL) contenant de l'hypophosphite de sodium (30 g), de l'acide citrique (CTR, 100 g) et la cyclodextrine ou la maltodextrine correspondante (100 g) est préparée à température ambiante sous agitation. Le solide polymérisé obtenu est dispersé dans de l'eau (1000 mL) et filtré sur fritté pour séparer la fraction soluble et de la fraction insoluble du polymère. La fraction insoluble est lavée à l'eau puis séchée à l'étuve (90°C) et conservée au sec et à l'abri de la lumière. Le filtrat est concentré à l'évaporateur rotatif et dialysé (6000 - 8000 MWCO) dans l'eau (2000 mL renouvelée toutes les 12 heures pendant 3 jours) ou purifié par ultrafiltration (Pellicon Appartus, 8000 MWCO, Millipore). Finalement, le dialysat contenant le polymère purifié est atomisé (B-290, Büchi, Suisse) ou lyophilisé (Christ(R)) pour obtenir une poudre blanche stockée au sec et à l'abri de la lumière. Le rendement de chaque fraction (soluble et insoluble) est déterminé par le ratio massique des poudres obtenues sur la masse des réactifs (CTR et cyclodextrines).

Dans le cas des polymères préparés avec deux cyclodextrines, la masse de chacune des cyclodextrines est de 50 g. La solution est séchée dans un évaporateur (Rotavapor, Büchi, Suisse) puis placée sous vide (60 mbar) à 140°C sur un bain d'huile pendant 90 minutes. Le solide polymérisé obtenu est dispersé dans de l'eau (1000 mL) et filtré sur fritté pour séparer la fraction soluble et de la fraction insoluble du polymère. La fraction insoluble est lavée à l'eau puis séchée à l'étuve (90°C) et conservée au sec et à l'abri de la lumière. Le filtrat est concentré à l'évaporateur rotatif et dialysé (6000 - 8000 MWCO) dans l'eau (2000 mL renouvelée toutes les 12 heures pendant 3 jours) ou purifié par ultrafiltration (Pellicon Appartus, 8000 MWCO, Millipore). Finalement, le dialysat contenant le polymère purifié est atomisé (B-290, Büchi, Suisse) ou lyophilisé (Christ(R)) pour obtenir une poudre blanche stockée au sec et à l'abri de la lumière. Le rendement de chaque fraction (soluble et insoluble) est déterminé par le ratio massique des poudres obtenues sur la masse des réactifs (CTR et cyclodextrines).

Tableau 1 : Caractéristiques de polymères de cyclodextrine synthétisés (le polymère "PolyCTR-Maltodextrine" ne tombe pas sous l'étendue de la protection revendiquée).

| POLYMÈRE | INSOLUBLE dry (g) | INSOLUBLE Q (%) * | INSOLUBLE YIELD (%) | SOLUBLE dry (g) | SOLUBLE YIELD (%) |
|---|---|---|---|---|---|
| PolyCTR-βCD | 223,6 | 282,1 | 32 | 79,6 | 11 |
| PolyCTR-HPβCD 1 | 180,6 | 353,6 | 26 | 114,4 | 16 |
| PolyCTR-HPβCD 2 | 400,8 | 241,7 | 57 | 66,1 | 9 |
| PolyCTR-MeβCD | 50 | 977,8 | 7 | 110,4 | 16 |
| PolyCTR-γCD | 128,9 | 156,8 | 18 | 94,7 | 13 |
| PolyCTR-HPγCD | 139,9 | 334,6 | 20 | 191,3 | 27 |
| PolyCTR-Maltodextrine | 416,8 | 290,2 | 59 | 58,3 | 8 |

Le tableau 1 montre le rendement en fraction soluble et insoluble obtenu lors de la synthèse des différents polymères de cyclodextrine. Les rendements globaux (insoluble + soluble) vont de 23% (PolyCTR-MebCD) à plus de 65% (PolyCTR-HPβCD 1 ; PolyCTR-MD) avec un rendement de la fraction insoluble plus élevé que celui de la fraction soluble; Néanmoins, il faut noter que les rendements et le ratio fraction soluble/insoluble peut varier en fonction des paramètres de synthèse.

La masse molaire des polymères solubles est déterminée par Chromatographie par perméation de gel (GPC) à l'aide d'un système DIONEX Ultimate 3000 équipé d'un détecteur miniDAWN TREOS de diffusion de la lumière à 3 angles (détecteur MALS, WYATT) et d'un réfractomètre différentiel SCHRAMBECK 2000. La longueur d'onde du faisceau laser du MALS est de 658 nm et les 3 angles de diffusion sont de 41,5, 90 et 138,5°C. Une colonne (SHODEX SB 806M HQ, 8 mm $\times$ 300 mm) et une pré-colonne (SHODEX OH-PAK SB-G, 6 mm $\times$ 50 mm) sont montés en série. La phase mobile est composée de tampon phosphate (0,15 mol/L), NaCl (1 mol/L) et NaN$_3$ (0,03%), le pH final est de 7,4. La vitesse d'élution de la colonne est de 0,8 mL/min. L'incrément d'indice dn/dc de 0,15 a été mesuré pour tous les polymères anioniques synthétisés ci-dessus. Les polymères sont dissous dans le tampon à une concentration de 4,5 g/L, filtré à 0,45 $\mu$m (Millipore) et stockés une nuit avant l'injection.

Le tableau 2 montre des exemples de synthèses du polymère de cyclodextrine, avec comme variables : la nature de la CD polymérisée, la durée et la température de réaction. En fonction de ces paramètres, on observe une variation des rendements réactionnels en fractions de polymères solubles et insolubles récupérées, ainsi qu'une variation des masses molaires des fractions de polymères solubles (mesurées par chromatographie par perméation de gel).

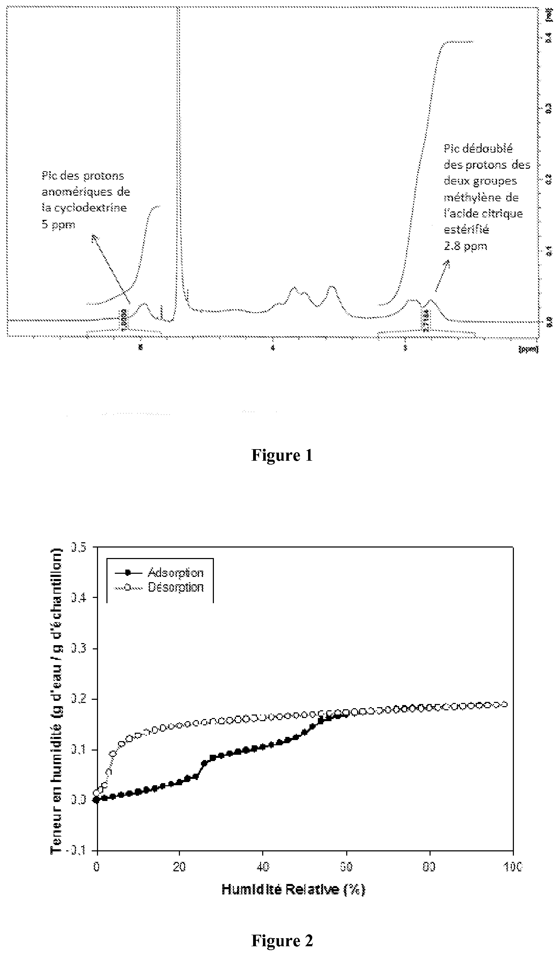

La teneur en cyclodextrine de la fraction soluble du polymère de cyclodextrine selon la présente invention peut être déterminée par résonance magnétique nucléaire (RMN). La fraction soluble du polyCTR-βCD a été caractérisée par RMN du proton au moyen d'un spectromètre Bruker Avance 300 MHz en solution dans l'oxyde de deutérium (figure 1). La figure 1 montre le spectre RMN $^1$H d'une fraction soluble de polymère où apparaissent d'une part le signal des deux groupes méthylènes (CH$_2$) de l'agent réticulant (acide citrique estérifié dont le déplacement chimique est d'environ 2,8 ppm (partie par million), et d'autre part le signal du proton H1 anomérique des unités glucoses de la cyclodextrine dont le déplacement chimique est à 5 ppm. L'intensité relative du premier pic est de 2,7184, tandis que celle du second pic est de 1,00. À partir de ces deux intensités, on peut calculer la composition massique du polymère en monomère de cyclodextrine (tableau 2) [Martel et al, Green Chem, 17, 2444-2454, 2015].

Tableau 2

| type CD (monomère) | Poids réactifs : acide citrique/ sodium hypophosphite/ CD introduits (en g) | température de réaction (°C) | durée de réaction (minutes) | poids fraction soluble du polymère (g) | poids fraction insoluble du polymère (g) | masse molaire fraction soluble (g/mol) | Pourcentage massique de CD mesuré par RMN |
|---|---|---|---|---|---|---|---|
| β | 250/75/250 | 140 | 40 | 45 | 25 | 9800 | 63% |
| β | 350/105/350 | 140 | 60 | 74 | 85 | 25500 | 60% |
| β | 350/105/350 | 140 | 100 | 82 | 224 | 35450 | 65% |
| HPγCD | 350/105/350 | 140 | 90 | 191 | 139 | 50000 | 65% |
| HPβCD | 350/105/350 | 140 | 90 | 114 | 180 | 64500 | 67% |

**Mesure de l'hygroscopie.**

Les mesures d'absorption et de désorption d'eau des polymères ont été réalisées à l'aide d'un analyseur thermogravimétrique SA Q5000 de TA Instruments contenant une microbalance dans lequel des creusets contenant l'échantillon et la référence ont été enfermés dans une chambre à humidité et température contrôlées. La température dans le Q5000 SA a été contrôlée par des éléments Peltier. Un gaz sec de $N_2$ (200 mL/min) a été divisé en deux parties, dont une partie a été humidifiée par passage dans une chambre saturée d'eau. L'humidité relative souhaitée (RH) pour les mesures peut ainsi être obtenue par mélange des proportions appropriées (régulées par les contrôleurs de débit massique) de flux sec et humide.

La mesure de l'isotherme standard est composée d'un certain nombre d'étapes. Premièrement, l'échantillon a été séché à 60°C et 0% d'humidité relative pendant un temps déterminé jusqu'à ce que la variation de poids soit stabilisée à moins de 0,05%. Dans un second temps, la température a été abaissée à 25°C. L'humidité a ensuite été augmentée progressivement (par pas de 2% d'humidité relative) à un maximum de 98% d'humidité relative. La variation de poids de l'échantillon a été stabilisée après chaque étape jusqu'à ce qu'elle soit inférieure à 0,05%. L'isotherme de désorption de l'eau a également été mesurée. Dans ce cas, le taux d'humidité a été réduit jusqu'à 0% (par pas de 2% d'humidité relative) et les échantillons ont été stabilisés en poids après chaque étape. Afin de finaliser l'isotherme et de comparer les résultats avec le poids initial de l'échantillon, une étape de séchage supplémentaire a été incluse (60°C à 0% d'humidité relative).

Concernant la βCD (figure 2), la rupture de la pente d'adsorption caractérise une hydratation qui se produit par 2 paliers successifs, témoignant d'une inclusion de molécules d'eau à l'intérieur de la cavité, et d'une hydratation des cristaux, phénomène bien connu de la littérature [Comprehensive supramolecular chemistry, Volume 3 Cyclodextrins, Volume Editors : Jozsef Szejtli & Tetsuo Osa, Pergamon, New York 1996]. L'isotherme de désorption d'eau ne se superpose pas à l'isotherme d'absorption révélant un phénomène d'hystérésis. Ceci indique que l'hydrate de cyclodextrine formé aux forts degrés hygrométriques est stable (10,5-12 molécules d'eau), et qu'il faut une atmosphère très sèche (en deçà de 20% RH) pour parvenir à désorber ces molécules d'eau, et à diminuer son degré d'hydratation la βCD.

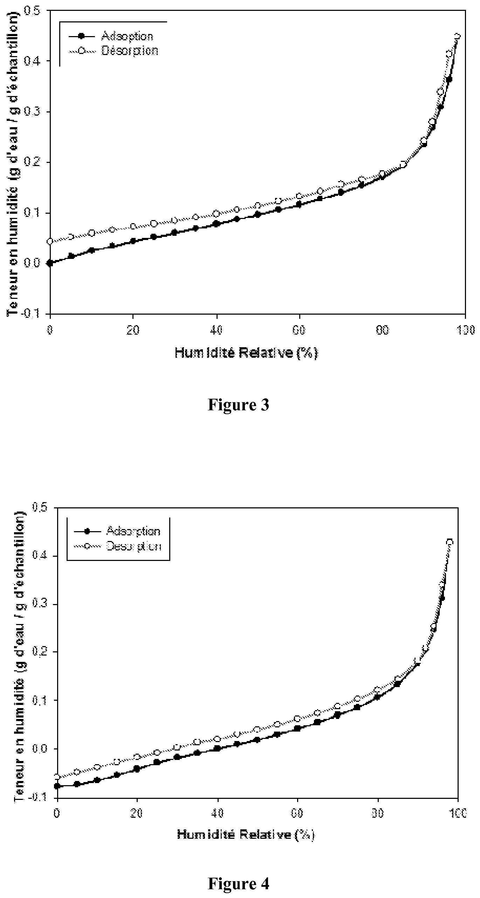

En revanche, l'analyse des courbes d'isothermes d'absorption de vapeur d'eau des deux polymères de βCD, soluble (figure 3) et insoluble (figure 4) montre d'une part une prise d'humidité régulière jusque 80%, qui devient ensuite exponentielle au-delà, jusque 98% d'humidité. La valeur maximale de prise d'eau atteinte pour les deux polymères est d'environ 0,45 g d'eau par gramme d'échantillon, ce qui est une valeur beaucoup plus élevée que celle mesurée pour la βCD (0,18 g/g). Ceci indique que le polymère de CD, soluble et insoluble présente une hydrophilie très supérieure à celle de la βCD. D'autre part, la courbe d'isotherme de désorption est presque superposée à celle de l'isotherme d'adsorption. Ceci indique que les molécules d'eau liées au polymère lors de la phase d'hydratation sont facilement éliminées lors de l'assèchement de l'atmosphère ambiante, par un processus d'équilibre. Cela indique qu'il est plus facile de contrôler le taux d'humidité du polymère que celui du monomère de βCD. Cette information est importante par rapport au paramètre du conditionnement des comprimés.

**Répartition granulométrique par diffraction laser (MasterSizer)**

La distribution de la taille des particules de la poudre séchée par atomisation de la fraction soluble du polyCTR-βCD a été mesurée avec un Mastersizer S (Malvern Instruments, Orsay, France) en utilisant une lentille de 300 mm. L'échantillon a été dispersé à l'état sec avec de l'air comprimé à 4 bar.

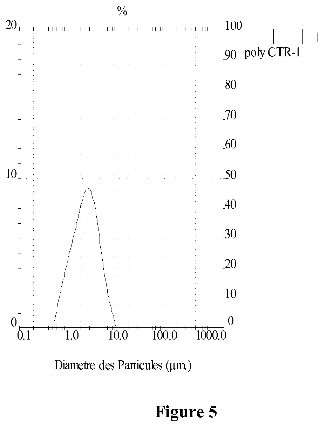

Ce polymère obtenu par atomisation présente un diamètre moyen de particule de 2,89 $\mu$m (figure 5).

**Clichés de poudre de poly-βCD atomisé et de poudre de poly-βCD lyophilisé**

Les clichés ont été pris à l'aide du microscope optique Olympus BX41 à des grossissements 20x et 50x. Le traitement des clichés a été réalisé à l'aide d'ImagePro Express 6.0.

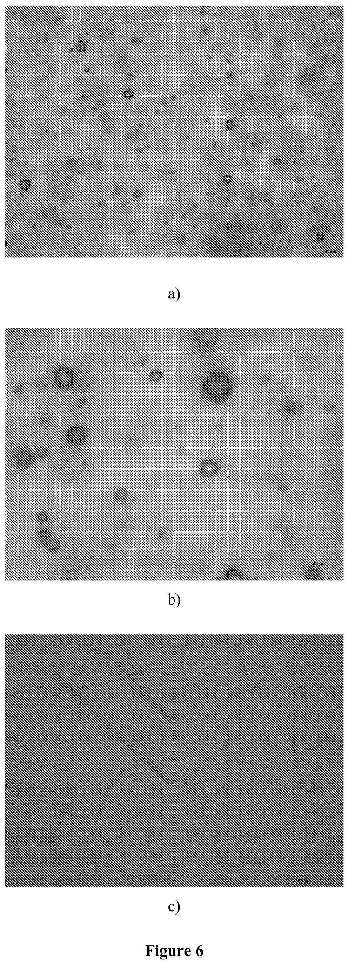

Les clichés pris au microscope optique mettent bien en évidence la différence structurale du poly-βCD obtenu par lyophilisation ou par atomisation. Par atomisation, le poly-βCD se présente sous la forme de microsphère de 1 à 25 $\mu$m (figure 6 (a) et figure 6 (b)) conforme aux données fournies par le constructeur. Par lyophilisation (figure 6 (c)), le polymère se présente sous forme d'aiguilles de plusieurs centaines de microns, forme défavorable à l'écoulement, à la régularité de remplissage de la matrice et à l'isotropie de la compression. Ces propriétés défavorables sont renforcées par la très faible densité des particules avec présence d'une quantité importante d'air interparticulaire dans le lit de poudre.

**Exemple 2 : Analyse de la solubilisation de principes actifs**

Les diagrammes de solubilité de plusieurs principes actifs ont été effectués en présence de cyclodextrines et de fractions solubles de polymères de cyclodextrine mentionnés à l'exemple 1.

Deux principes actifs ont ainsi été testés : l'ibuprofène et le piroxicam.

Des solutions de concentration croissante de cyclodextrines et fractions solubles de polymères de cyclodextrine ont été préparées dans de l'eau ultrapure [0-70 g/L]. 10 mL de chacune des solutions ont été mis en présence d'un excès du principe actif (20 mg) sous agitation pendant 24 heures à température ambiante. Les solutions ont ensuite été filtrées avec une membrane en cellulose et en Nylon (0,45 $\mu$m; VWR International, USA) respectivement pour l'Ibuprofène et le Piroxicam. L'ibuprofène a été dosé par HPLC (Shimadzu LC-2010 HT Liquid Chromatography), la phase mobile de l'ibuprofène est un mélange acétonitrile/eau (60:40) avec un pH de 2,25, une colonne C18 a été utilisée. Le piroxicam a été dosé par spectrophotométrie UV-Vis à 356 nm (UV 1800 Shimadzu).

La constante de stabilité ($K_{1:1}$) est utilisée pour comparer l'affinité de principes actifs pour différentes cyclodextrines ou dérivés. La solubilité totale de médicament ($S_t$) en solution aqueuse de cyclodextrine sera :

$$St = So + \frac{K\,1{:}1\,x\,So}{1 + (K\,1{:}1\,x\,So)}\,x\,[CD]t \quad (1)$$

$S_o$ = solubilité intrinsèque du médicament; $[CD]_1$ = concentration totale en cyclodextrine dans le milieu aqueux

Une parcelle de St rapport [CD] t, selon l'équation (1), donnera une ligne droite avec une pente ($K_{1:1}\,S_o$ / (1 + $K_{1:1}\,S_o$)) et une interception ($S_{int}$) égale à $S_o$. $K_{1:1}$ est calculée à partir de la pente et $S_o$ :

$$K\,1{:}1 = \frac{Slope}{So\,(1 - Slope)} \quad (2)$$

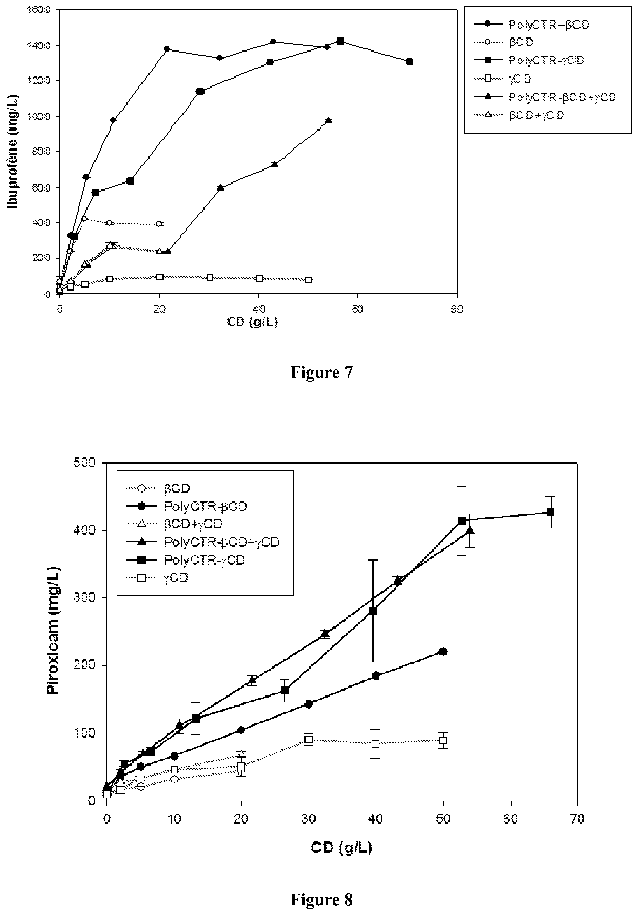

Comme représenté à la figure 7, la solubilité de l'ibuprofène en présence des fractions solubles de polymères de cyclodextrine est largement supérieure, en comparaison aux cyclodextrines respectives. Ainsi, la $\gamma$CD par exemple permet de solubiliser moins de 100 mg/L d'ibuprofène quelle que soit la concentration en $\gamma$CD (0-50 g/L), alors que le PolyCTR-$\gamma$CD permet de solubiliser jusqu'à 1500 mg/L pour une concentration en polymère de cyclodextrine de 50 g/L. Autre point intéressant, alors que la limite de solubilité de la $\beta$CD est de 20 g/L, la solubilité du PolyCTR-$\beta$CD est d'au moins 70 g/L. Tout comme dans le cas de la $\gamma$CD, la fraction soluble du polymère de cyclodextrine permet de sensiblement améliorer la solubilité de l'ibuprofène (400 mg/L vs 1400 mg/L pour une concentration de 20 g/L de $\beta$CD et de PolyCTR-$\beta$CD respectivement). Finalement la fraction soluble d'un polymère contenant deux cyclodextrines ($\beta$CD et $\gamma$CD) permet également d'améliorer la solubilité de l'ibuprofène sans toutefois obtenir les même performances (1000 mg/L vs 1400 mg/L respectivement pour le polymère contenant les deux cyclodextrines (50 g/L) et le polymère ne contenant qu'une cyclodextrine (50 g/L)).

Les diagrammes de solubilité ont également été réalisés avec d'autres cyclodextrines et la fraction soluble de leurs polymères synthétisés. Les données en termes de solubilisation de l'ibuprofène avec une concentration de 10 et 50 g/L en cyclodextrine ainsi que les constantes de complexation associées sont données dans le tableau 3.

Afin de pouvoir faire une comparaison directe entre cyclodextrine monomère et cyclodextrine polymérisée, les diagrammes de solubilité sont présentés en fonction de la concentration rapportée en gramme de monomère de cyclodextrine par litre.

Tableau 3 : Solubilité de l'ibuprofène et constantes de complexation en présence des cyclodextrines ou de fractions solubles de polymères de cyclodextrine à 10 et 50 g/L en cyclodextrine. L'utilisation de cyclodextrines sous forme de monomères ne tombe pas sous l'étendue de la protection revendiquée.

| Agent de solubilisation | Solubilité à 10 g/L (mg/L) | Solubilité à 50 g/L (mg/L) | Constante de complexation $K_{1:1}$, M$^{-1}$ |
|---|---|---|---|
| $\beta$CD | 400 | / | 11 |
| HP$\beta$CD1 | 700 | 1100 | 17 |
| HP$\beta$CD2 | 700 | 1100 | 45 |
| Me$\beta$CD | 700 | 1200 | 33 |
| $\gamma$CD | 100 | 100 | 1 |
| HP$\gamma$CD | 700 | 1200 | 11 |
| PolyCTR-$\beta$CD | 1000 | 1400 | 18336 |
| PolyCTR-HP$\beta$CD1 | 800 | 1400 | 19714 |
| PolyCTR-HP$\beta$CD2 | 800 | 1400 | 17561 |

(suite)

| Agent de solubilisation | Solubilité à 10 g/L (mg/L) | Solubilité à 50 g/L (mg/L) | Constante de complexation $K_{1:1}$, $M^{-1}$ |
|---|---|---|---|
| PolyCTR-MeβCD | 800 | 1400 | 13672 |
| PolyCTR-γCD | 600 | 1400 | 3381 |
| PolyCTR-HPγCD | 700 | 800 | 6278 |
| PolyCTR-βCD/γCD | 300 | 1000 | 1385 |
| PolyCTR-HPβCD/HPγCD | 200 | 600 | 3073 |

Le tableau 3 montre que toutes les cyclodextrines permettent de solubiliser jusqu'à 1000 mg/L d'ibuprofène alors que les fractions solubles des polymères permettent de solubiliser jusqu'à 1400 mg/L d'ibuprofène.

Comme représenté à la figure 8, la solubilité du piroxicam en présence de fraction soluble de PolyCTR-βCD est bien supérieure à la solubilité du piroxicam en présence de β-CD, cette même interprétation peut être réalisée avec la γCD et le polyCTR-γCD. Dans le cas du piroxicam, le PolyCTR-γCD semble améliorer sensiblement la solubilité comparé au PolyCTR-βCD (400 mg/L vs 200 mg/L à une concentration de 50 g/L en polymère). La fraction soluble du polymère composé de deux cyclodextrines (βCD et γCD) permet également de solubiliser efficacement le piroxicam (400 mg/L à une concentration en polymère de 50 g/L).

Les diagrammes de solubilité ont également été réalisés avec d'autres cyclodextrines et les fractions solubles de leurs polymères synthétisés. Les données en termes de solubilisation du piroxicam avec une concentration en cyclodextrines ou de fractions solubles de polymères de cyclodextrine de 10 et 50 g/L en cyclodextrine ainsi que les constantes de complexation associées sont données dans le tableau 4.

Tableau 4 : Solubilité du piroxicam et constantes de complexation en présence de cyclodextrines ou de fraction soluble de polymère de cyclodextrine à 10 et 50 g/L. L'utilisation de cyclodextrines sous forme de monomères ne tombe pas sous l'étendue de la protection revendiquée.

| Agent de solubilisation | Solubilité à 10 g/L (mg/L) | Solubilité à 50 g/L (mg/L) | Constante de complexation $K_{1:1}$, $M^{-1}$ |
|---|---|---|---|
| βCD | 30 | / | 173 |
| HPβCD2 | 30 | 50 | 185 |
| γCD | 40 | 70 | 890 |
| PolyCTR-βCD | 70 | 200 | 787 |
| PolyCTR-tIPPCD2 | 70 | 150 | 192 |
| PolyCTR-γCD | 100 | 400 | 1133 |
| PolyCTR-HPβCD/HPγCD | 70 | 150 | 257 |

Le tableau 4 montre que toutes les cyclodextrines permettent de solubiliser jusqu'à 100 mg/L de piroxicam alors que les fractions solubles des polymères permettent de solubiliser jusqu'à 400 mg/L de piroxicam avec un avantage certain pour la γCD et le PolyCTR-γCD.

**Exemple 3** : Granulation humide d'une fraction soluble d'un polymère de cyclodextrine et de principes actifs ainsi que de cyclodextrines et de principes actifs.

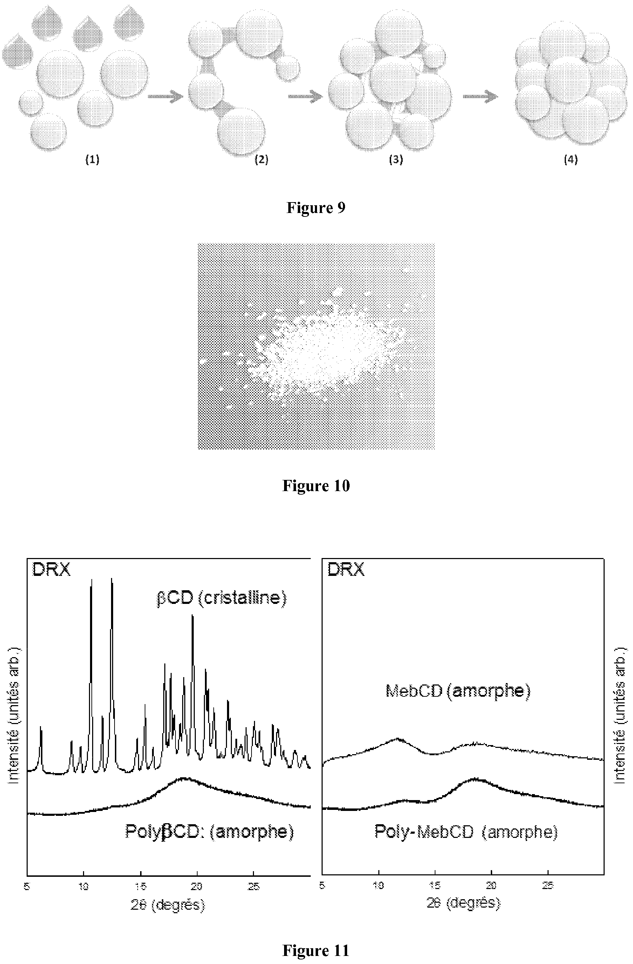

La granulation humide (Figure 9) a été réalisée en mélangeant la cyclodextrine ou la fraction soluble de polymère de cyclodextrine avec le principe actif et de l'eau (liant) en quantité suffisante pour former une masse humide. Dans le cas de l'ibuprofène le ratio massique CD (ou polyCD)/Ibuprofène (90/10) a été choisi, il était de 60/40 dans le cas du piroxicam. L'eau a été ajoutée à la micropipette par portion de 100 μL successivement jusqu'à l'obtention d'une masse humide d'humidité suffisante. La préparation a été séchée à 60°C pendant 10 minutes puis granulée pour obtenir une taille de particule de 630 μm.

Plusieurs granulations humides ont été effectuées en utilisant la βCD, γCD, HPβCD1, HPβCD2, HPγCD et MeβCD ou les fractions solubles des polymères de cyclodextrine correspondants en mélange avec l'ibuprofène ou le piroxicam. Dans tous les cas une poudre blanche a été obtenue avec une taille de particule de 630 μm (figure 10).

**Exemple 4 : Caractérisation physique**

CARACTERISATION STRUCTURALE DES FRACTIONS SOLUBLES DE POLY-βCD ET DE POLY-MebCD (DRX)

Les investigations par diffraction des rayons X ont été réalisées avec le diffractomètre XPERT PRO MPD de chez Panalytical ($\lambda_{CuK\alpha}$ = 1.540 Å) équipé du détecteur "X'celerator". Les échantillons ont été placés dans un capillaire de Lindemann de diamètre 0,7 mm et les mesures ont été réalisées à 20°C.

La figure 11 montre les diagrammes de diffractions des rayons X de la poly-βCD et de la polyMebCD. Les diagrammes des cyclodextrines correspondantes sont également reportés pour comparaison. On constate que les diagrammes de la poly-βCD et de la poly-MebCD ont la forme d'un halo diffus et ne présentent aucune raie de Bragg. La poly-βCD et la poly-MebCD sont donc des matériaux amorphes. Les diagrammes des monomères correspondants indiquent que la βCD est cristalline (présence de pics de Bragg) alors que la MebCD (méthyl-bétacyclodextrine, d'appellation commerciale Crysmeb) est amorphe (absence de pic de Bragg).

CARACTERISATION DE LA TRANSITION VITREUSE DES FRACTIONS SOLUBLES DE POLY-βCD ET DE POLY-MebCD (DSC)

Les expériences de calorimétrie ont été réalisées avec la DSC Q200 de chez TA Instruments. Les échantillons ont été placés dans des creusets en aluminium ouverts et balayés avec de l'azote gazeux pur. L'instrument a été étalonné en température et en enthalpie en utilisant l'indium comme standard. Des mélanges amorphes homogènes poly-βCD / mannitol de concentration en mannitol (X) variant de 30 à 80 % ont été préparés par fusion-trempe des mélanges physiques correspondants à 200°C.

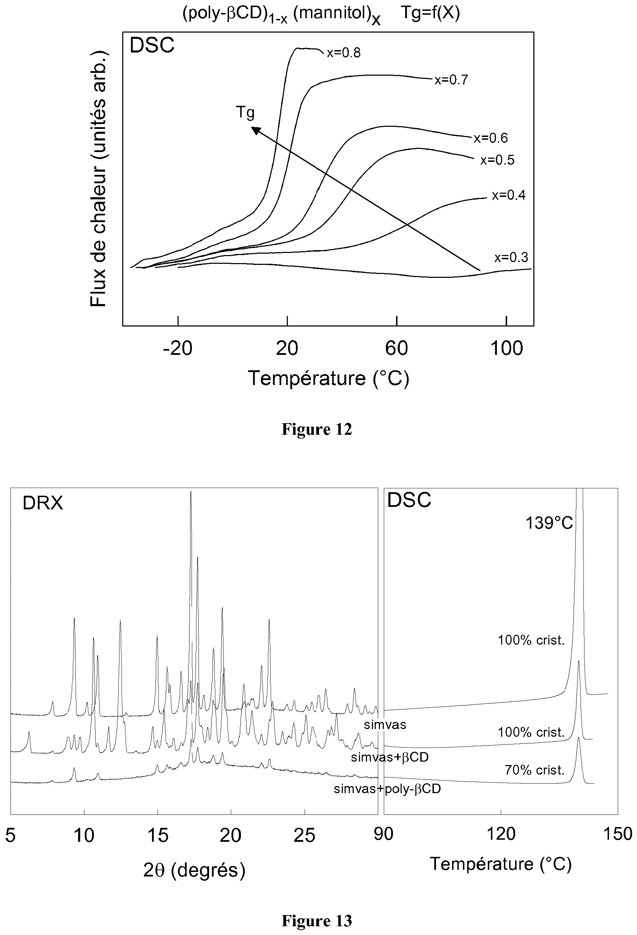

La figure 12 montre les thermogrammes des mélanges poly-βCD / mannitol enregistrés au chauffage (5°C/min). Ils présentent tous un saut de chaleur spécifique (Cp) caractéristique d'une transition vitreuse. Ce saut est unique, ce qui indique que les mélanges sont dans un état physique amorphe homogène. La température de transition vitreuse Tg (prise au point d'inflexion du saut) diminue lorsque la concentration en mannitol augmente ce qui montre l'effet plastifiant du mannitol sur la poly-βCD. L'évolution du Tg du mélange ave la concentration en mannitol (X) suit une loi de Gordon Taylor dont l'extrapolation à X = 0 permet d'estimer le Tg de la poly-βCD pure. On trouve Tg # 400°C. La transition vitreuse de la poly-βCD se situe donc à très haute température et bien au-dessus de sa température de dégradation thermique. Elle ne peut donc pas être observée directement en DSC. Une étude analogue menée avec la poly-MebCD révèle un comportement identique et une transition vitreuse proche de Tg = 317°C.

CARACTERISATION STRUCTURALE D'UNE FORMULATION FRACTION SOLUBLE DE POLY-βCD / SIMVASTATINE OBTENUE PAR GRANULATION

La figure 13 montre les diagrammes de diffraction des rayons X enregistrés à 20°C et les thermogrammes enregistrés au chauffage (5°C/min) de la simvastatine, d'un mélange simvastatine / poly-βCD et d'un mélange simvastatine / βCD. Les deux mélanges ont été obtenus par granulation humide et la concentration de simvastatine est de 10%. Les diffractogrammes des mélanges présentent clairement des raies de Bragg caratéristiques de la simvastatine cristalline. Les thermogrammes présentent, quant à eux, un endotherme caractéristique de la fusion de la simvastatine cristalline. Ces deux observations indiquent que chaque formulation contient une fraction de simvastatine cristalline. L'analyse des enthalpies de fusion indique cependant que cette fraction cristalline est plus importante dans la formulation à base de βCD que dans celle à base de poly-βCD. Cela indique que la poly-βCD permet de réduire "sensiblement" la cristallinité de la simvastatine, par rapport au mélange équivalent à base de βCD monomère.

FORMATION D'UNE SOLUTION VITREUSE PIROXICAM POLY-βCD / PIROXICAM

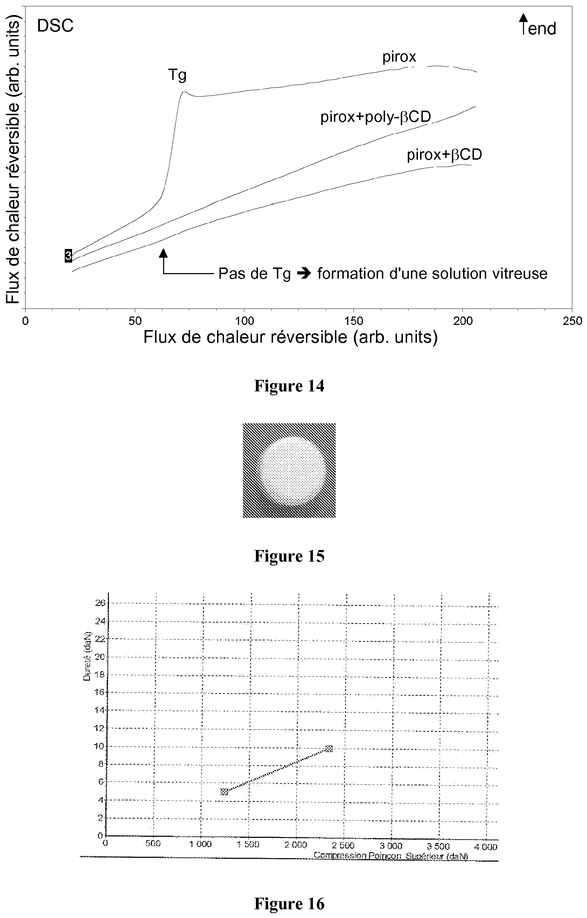

Un échantillon de piroxicam pur, un mélange physique piroxicam/poly-βCD (1:9) et un mélange physique piroxicam/βCD (1:9) ont été chauffés (5°C/min) jusqu'à 210°C, c'est-à-dire au-dessus de la température de fusion du piroxicam ($T_m$ = 201°C). Ils ont ensuite été refroidis à 20°C. La figure 14 montre les thermogrammes enregistrés au chauffage (5°C/min) de ces échantillons trempés. Le piroxicam montre clairement un saut de chaleur spécifique à Tg = 65°C qui indique que le piroxicam est sous une forme amorphe. Les deux mélanges ne montrent par contre aucun signe de transition vitreuse à cette température. Ils ne montrent pas non plus d'endotherme de fusion à 201°C. Ces faits indiquent que ces mélanges ne contiennent ni piroxicam en phase amorphe ni piroxicam en phase cristalline. Les molécules de piroxicam sont en fait dispersées à l'échelle moléculaire dans la matrice de poly-βCD ou de βCD, formant des « solutions vitreuses ». NB : On n'observe aucune trace des transitions vitreuses de ces mélanges qui sont attendues à des températures supérieures à 210°C en raison des Tg très élevées de la βCD et de la poly-βCD et de la faible proportion de piroxicam (10%) dans les deux mélanges. La poly-βCD et le piroxicam peuvent donc former des solutions vitreuses homogènes de grande stabilité physique.

**Exemple 5 : Préparation de comprimés**

La compression est une étape qui permet de densifier la poudre afin de la mettre sous forme de comprimés. Le poinçon supérieur de la presse coule dans la matrice. Ce puits est contrôlé par une force ou par un déplacement imposé. Au début du compactage, les particules sont réarrangées par translation et rotation pour former un empilement dense. Le lit de poudre est ensuite débarrassé de l'excès d'air et le nombre de points de contact entre les particules augmente. À la fin de l'étape de compactage, les particules ne peuvent pas glisser et il existe une réelle résistance de la poudre à la pression du poinçon. Les particules subissent des déformations en fonction de leur comportement mécanique. Les particules fragiles auront tendance à se fragmenter ce qui entraîne un nouveau réarrangement et une plus grande densification locale. Au cours de la fragmentation, de nouvelles particules sont formées et le processus suivant commence : réarrangement, déformation réversible et irréversible, enchevêtrement et fragmentation à une taille critique minimale. La plupart des particules ductiles se déforment de manière irréversible permettant de maintenir les liaisons entre les particules et la cohésion des comprimés.

Matériels et méthodes

**Préparation de comprimés**

La machine à comprimer alternative utilisée était une Korsch (Allemagne) équipé de poinçon plat cylindrique de diamètre 5 mm. Les étapes de compression étaient les suivantes: (1) le remplissage de la matrice par écoulement du mélange, (2) arasage, (3) compression et (4) éjection du comprimé. L'épaisseur moyenne des comprimés est de 3,2 mm.

**Caractérisation des comprimés**

*Résistance à la rupture*

Cet essai est destiné à déterminer, dans des conditions définies, la résistance à la rupture des comprimés, mesurée par la force nécessaire pour les rompre par écrasement entre deux mâchoires.

L'appareil utilisé (PTB 311E, Pharma test Instruments, Inde) est constitué de deux mâchoires se faisant face, dont l'une se déplace vers l'autre qui est fixe. Les surfaces planes des mâchoires sont perpendiculaires à la direction de déplacement.

Le mode opératoire consistait à mettre en place le comprimé à plat entre les mâchoires, puis, une mâchoire se déplaçait à vitesse constante pour appliquer une force jusqu'à la rupture du comprimé. Cette force maximale est notée et considérée comme la dureté du comprimé. La façon dont un comprimé se fracture quand une force croissante est appliquée varie considérablement selon les diverses caractéristiques des produits.

*Temps de Désintégration*

L'appareil utilisé était un appareil de désintégration manuelle PTZ-S, Pharma Test Instruments (Inde), composé d'un panier porte-tubes, un vase cylindrique bas de 1000 mL destiné à contenir le fluide d'immersion, un agencement thermostatique pour chauffer le fluide entre 35°C et 39°C, et d'un dispositif servant à imprimer au porte-tubes, dans le liquide d'immersion, un mouvement vertical alternatif. Le panier est composé de six tubes transparents verticaux, ouverts à leur extrémité supérieure et fermés à l'extrémité inférieure par une grille, un comprimé était placé dans chacun des six tubes du panier. Le fluide d'immersion était l'eau.

Le temps de désintégration a été déterminé comme le temps nécessaire pour que les comprimés passent à travers les mailles de la grille.

*Densité après tassement et indice de compressibilité de la composition compressible.*

L'appareil utilisé est un appareil de tassement (Engelsmann powder tester, PTTD200, Pharma Test Instruments, India) composé d'une éprouvette graduée de 100 mL sur un socle vibrant. Les manipulations ont été réalisées suivant les conditions décrites dans la pharmacopée européenne (1997). Pour chaque poudre, le volume initial avant tassement est mesuré (V0) permettant de calculer la densité p0. Le volume final (Vf) de la poudre est ensuite mesuré après 500 tassements. La densité finale de la poudre après tassement peut ainsi être calculée (pf). On en déduit également l'indice de compressibilité ou Indice de Carr (Ic) donné par la formule suivante :

$$\mathrm{Ic}\ (\%) = \left(\rho f/\rho 0\right) \times 100$$

Les poudres ayant un indice de Carr inférieur à 20% présentent un bon écoulement.

Résultats

*Densités après tassement et indices de compressibilité*

Le tableau 5 présente la densité après tassement et les indices de Carr de la βCD, de la fraction soluble du polymère de βCD et du mélange à différents ratios de la fraction soluble et de la fraction insoluble du polymère de βCD.

Le tableau 5 montre que la βCD et la fraction soluble de polymère de cyclodextrine obtenu par atomisation présentent un Ic > 20%; le même résultat est obtenu pour les mélanges de fractions insoluble et soluble du polymère présentant des ratios respectifs de 25%/75% à 80%/20%. Seuls les ratios 99%/1% et 90%/10% montrent un bon écoulement.

**Tableau 5.** L'utilisation de cyclodextrines sous forme de monomères ne tombe pas sous l'étendue de la protection revendiquée.

| Composition | Ecoulement | TAP (lc*) (%) | $\rho$f (g/mL) | Masse (g) | Dureté (N) |
|---|---|---|---|---|---|
| *100% Insoluble* | Libre | 11,1 | 0,501 | 0,101 | 16 |
| *99% Insoluble + 1% Soluble* | Libre | 14.3 | 0.500 | 0.5051 | |
| *90% Insoluble + 10% Soluble* | Libre | 16,7 | 0,502 | 0,0970 | 24 |
| *80% Insoluble + 20% Soluble* | Non Libre | 22 | 0,511 | 0,1047 | 52 |
| *75% Insoluble + 25% Soluble* | Non Libre | 23,5 | 0,526 | 0,1033 | 48 |
| *50% Insoluble + 50% Soluble* | Non Libre | 25 | 0,666 | | |
| *25% Insoluble + 75% Soluble* | Non Libre | 27,6 | 0,476 | 0,0970 | 87 |
| *100% Soluble* | Non Libre | 28,2 | 0,357 | 0,0937 | 64 |
| *100% soluble Granulation Humide* | Libre | 18 | 0,700 | | |
| *CD (monomère)* | Non Libre | 23,1 | 1,066 | | |

Le tableau 5 montre également qu'après une étape de granulation humide, la fraction soluble du polymère de cyclodextrine présente un bon écoulement. Cette étape permet donc en plus de favoriser l'inclusion d'un principe actif, une amélioration de l'écoulement de la poudre.

*Compression et caractérisation des comprimés*

Les comprimés obtenus par compression (Figure 15) étaient blancs, avec une surface lisse et un bon aspect dans tous les cas, un diamètre de 5 mm et une masse de 100 mg.

**Tableau 6.** Caractéristiques des comprimés de 100 mg. L'utilisation de cyclodextrines sous forme de monomères ne tombe pas sous l'étendue de la protection revendiquée.

| Composition | Résistance à la rupture (N) | Temps de désagrégation |
|---|---|---|
| *100 % Insoluble* | 60-70 | < 15 sec |
| *99% Insoluble + 1% Soluble* | 60-70 | 5 min |
| *90% Insoluble + 10% Soluble* | 40 | 8 min |
| *80% Insoluble + 20% Soluble* | 50 | 14 min |
| *75% Insoluble + 25% Soluble* | 60-70 | 15 min |

(suite)

| Composition | Résistance à la rupture (N) | Temps de désagrégation |
|---|---|---|
| *50% Insoluble + 50% Soluble* | 60-70 | 15 min |
| *25% Insoluble + 75% Soluble* | 30-40 | 15 min |
| *100% Soluble* | 30-40 | 14 min |
| *100% soluble Granulation Humide* | 60-70 | 15 min |
| *CD (monomère)* | 45-50 | > 30 min |

Les comprimés étudiés dans le tableau 6 ont été préparés uniquement à partir des fractions soluble et insoluble du polymère, sans ajouter d'autres additifs (agent d'écoulement, agent de désagrégation, enrobage ...), et sans principe actif. La résistance à la rupture de la fraction insoluble utilisée seule est de 60-70N, et celle de la fraction soluble atomisée seule est de 30-40 N ; une résistance à la rupture maximale est mesurée pour les mélanges des fractions insoluble/soluble 75:25 et 50:50. Le temps de désagrégation des comprimés (Tableau 6) à base de la fraction soluble atomisée est de 14 minutes, celui d'une fraction soluble traitée par granulation humide est de 15 minutes, tandis que celui relatif aux comprimés à base de fraction insoluble est inférieur à 15 secondes. Le tableau 6 montre que lorsque l'on mélange les fractions insoluble/soluble dans des proportions comprises entre 100:0 et 80:20, le temps de désagrégation varie en passant de moins de 15 secondes à 14 minutes.

*Dureté des comprimés obtenus par compression directe*

La fraction soluble a été comprimée par compression directe à l'aide d'une machine à comprimer alternative instrumentée Frogerais OA équipée de poinçons plats de diamètre 11,28 mm. La vitesse de la machine est de 1 comprimé par seconde. L'épaisseur des comprimés est de 2 mm. L'instrumentation couplée à un logiciel permet l'acquisition des phénomènes physiques de la compression.

La courbe dureté = f(force de compression) de la figure 16 montre qu'il est possible d'obtenir des duretés (résistances à la rupture) correctes (8 à 10 kN) sans addition d'excipient et pour des forces de compression très acceptables (2000 daN).

**Exemple 6 : Analyse de la dissolution de comprimés.** L'utilisation de cyclodextrines sous forme de monomères ne tombe pas sous l'étendue de la protection revendiquée.

Matériels et méthodes

La dissolution de principes actifs à partir de comprimés selon l'invention, i.e. les comprimés formés à partir de fractions solubles de polymères de cyclodextrine, a été comparée à la dissolution de principes actifs à partir de comprimés formés à partir de cyclodextrines sous forme de monomères ainsi qu'à la dissolution du principe actif non compris dans une forme galénique.

Pour cela, trois principes actifs ont ainsi été testés : l'ibuprofène, la simvastatine et le piroxicam.

La poudre contenant les cyclodextrines ou les fractions solubles de polymère de cyclodextrine et le principe actif a été obtenue par granulation humide comme détaillé dans l'exemple 4. Le ratio CD ou PolyCD / ibuprofène était de 90/10 et le ratio CD ou PolyCD / Piroxicam était de 60 / 40. Les essais de dissolution ont été réalisés dans un système Sotax® USP 4 (50 mL/min, 37°C) muni d'un détecteur UV. L'ibuprofène et le piroxicam ont été détectés à une longueur d'onde de 264 et 334 nm, respectivement.

Le milieu de dissolution est composé de 500 mL de HCl (pH = 1) dans le cas de l'ibuprofène, et de 500 mL de HCl (pH = 4) dans le cas du piroxicam. Des échantillons ont été prélevés toutes les 2 minutes pendant 1 h et analysés par un spectromètre UV-VIS (Perkin Elmer Lambda 25). Les courbes sont obtenues en pourcentage en divisant la quantité de principe actif libérée au cours du temps par la quantité de principe actif initiale.

Résultats

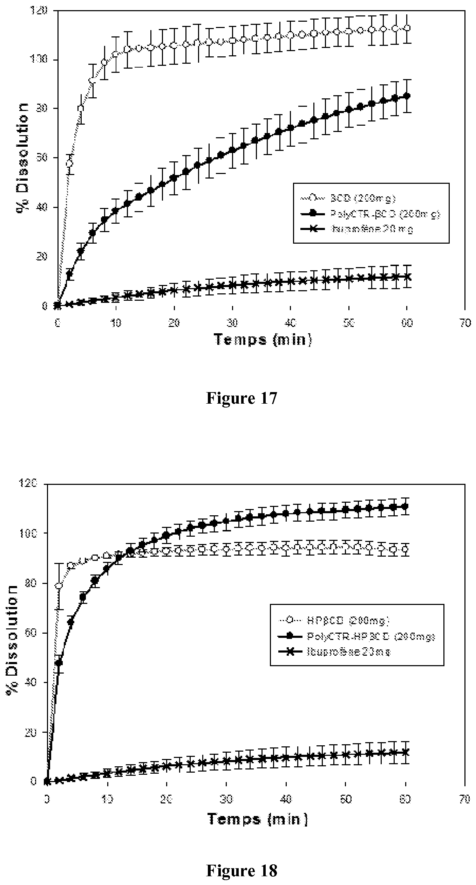

Les résultats avec l'ibuprofène (Figures 17, 18 et 19) montrent que quels que soient la cyclodextrine et les fractions solubles de polymère de cyclodextrine choisis, les comprimés sont conformes aux spécifications de la pharmacopée avec une dissolution d'au moins 75% du principe actif en moins de 45 minutes alors que l'ibuprofène se libère à hauteur de 15% en 45 minutes. Les cyclodextrines et la fraction soluble du polymère permettent donc d'améliorer la dissolution de l'ibuprofène. La fraction soluble du polymère permet quant à lui d'avoir un profil de libération contrôlée comparé aux cyclodextrines natives en évitant une libération brutale du principe actif dans les premières minutes (burst effect).

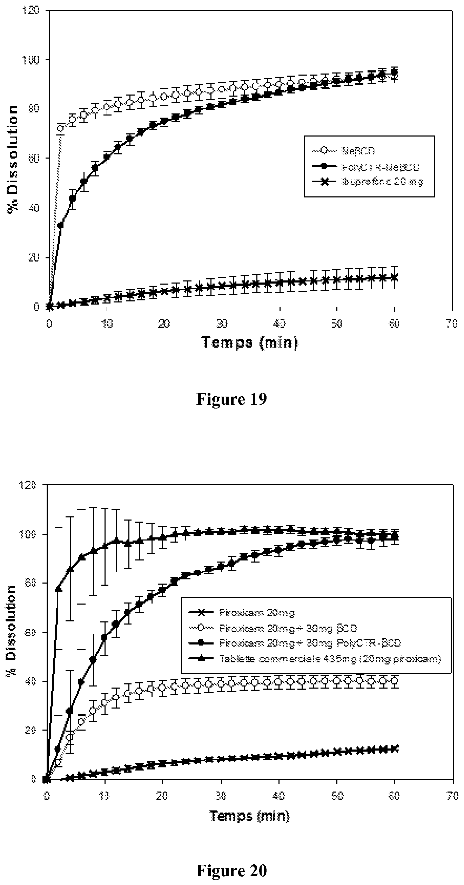

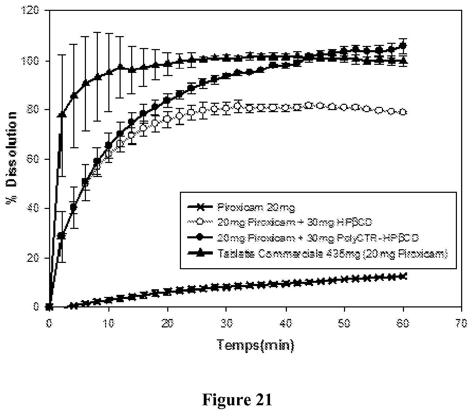

Les résultats présentés dans les Figures 20 et 21 montrent que le piroxicam seul ou dans la formulation commerciale se libère à hauteur de 10% et 100% respectivement en 45 minutes.

Une diminution de la quantité de cyclodextrine dans la formulation (30 mg au lieu de 415 mg) ne permet plus d'être conforme aux spécifications de la pharmacopée avec moins de 75% du principe actif libéré en 45 minutes. Par contre, pour cette même quantité d'excipient, les fractions solubles de polymères de cyclodextrine ($\beta$CD ou HP$\beta$CD) permettent de libérer 100% de l'ibuprofène en 45 minutes.

## Revendications

1.  Utilisation d'une fraction insoluble dans l'eau d'un polymère de cyclodextrine réticulé par un acide poly(carboxylique) obtenu par condensation des groupes hydroxyle d'une cyclodextrine avec les fonctions carboxyliques de l'acide poly(carboxylique), d'une fraction soluble dans l'eau d'un polymère de cyclodextrine réticulé par un acide poly(carboxylique) obtenu par condensation des groupes hydroxyle d'une cyclodextrine avec les fonctions carboxyliques de l'acide poly(carboxylique) ou d'un mélange de fractions insoluble dans l'eau et soluble dans l'eau, préalablement séparées puis remélangées, de polymères de cyclodextrine réticulés par un acide poly(carboxylique) obtenu par condensation des groupes hydroxyle d'une cyclodextrine avec les fonctions carboxyliques de l'acide poly(carboxylique) en tant qu'excipient unique dans un comprimé, dans laquelle l'acide poly(carboxylique) est l'acide citrique, la masse molaire moyenne en poids mesurée par chromatographie d'exclusion stérique de la fraction soluble dans l'eau du polymère de cyclodextrine est comprise entre 10 000 et 200 000 g.mol$^{-1}$.

2.  Utilisation selon la revendication 1, dans laquelle l'excipient est un excipient de compression, notamment de compression directe ou de compression après une étape de granulation humide, excipient de solubilisation et/ou excipient de complexation pour un comprimé.

3.  Utilisation selon la revendication 1 ou 2, dans laquelle on utilise une fraction insoluble dans l'eau d'un polymère de cyclodextrine.

4.  Utilisation selon la revendication 1 ou 2, dans laquelle on utilise une fraction soluble dans l'eau d'un polymère de cyclodextrine.

5.  Utilisation selon la revendication 4, dans laquelle la fraction soluble dans l'eau a été granulée par voie humide.

6.  Utilisation selon la revendication 1 ou 2, dans laquelle on utilise un mélange de fractions insoluble dans l'eau et soluble dans l'eau de polymères de cyclodextrine.

7.  Utilisation selon la revendication 6, dans laquelle le ratio fraction insoluble dans l'eau de polymère de cyclodextrine / fraction soluble dans l'eau de polymère de cyclodextrine est compris entre 99:1 et 80:20.

8.  Utilisation selon la revendication 6, dans laquelle le ratio fraction insoluble de polymère de cyclodextrine / fraction soluble de polymère de cyclodextrine est compris entre 80:20 et 25:75.

9.  Utilisation d'un mélange de fractions insoluble dans l'eau et soluble dans l'eau, préalablement séparées puis remélangées, de polymères de cyclodextrine réticulés par un acide poly(carboxylique) obtenu par condensation des groupes hydroxyle d'une cyclodextrine avec les fonctions carboxyliques de l'acide poly(carboxylique) en tant qu'excipient dans un comprimé, dans laquelle l'acide poly(carboxylique) est l'acide citrique, la masse molaire moyenne en poids mesurée par chromatographie d'exclusion stérique de la fraction soluble dans l'eau du polymère de cyclodextrine est comprise entre 10 000 et 200 000 g.mol$^{-1}$.

10. Utilisation selon la revendication 9, dans laquelle l'excipient est un excipient de compression, notamment de compression directe ou de compression après une étape de granulation humide, excipient de solubilisation et/ou excipient de complexation pour un comprimé.

11. Utilisation selon la revendication 10, dans laquelle le ratio fraction insoluble dans l'eau de polymère de cyclodextrine / fraction soluble dans l'eau de polymère de cyclodextrine est compris entre 99:1 et 80:20.

12. Utilisation selon la revendication 10, dans laquelle le ratio fraction insoluble de polymère de cyclodextrine / fraction soluble de polymère de cyclodextrine est compris entre 80:20 et 25:75.

13. Composition compressible constituée d'une fraction insoluble d'un polymère de cyclodextrine réticulé par un acide poly(carboxylique) obtenu par condensation des groupes hydroxyle d'une cyclodextrine avec les fonctions carboxyliques de l'acide poly(carboxylique), d'une fraction soluble d'un polymère de cyclodextrine réticulé par un acide poly(carboxylique) obtenu par condensation des groupes hydroxyle d'une cyclodextrine avec les fonctions carboxyliques de l'acide poly(carboxylique) ou d'un mélange de fractions insolubles et solubles de polymères de cyclodextrine réticulé par un acide poly(carboxylique) obtenu par condensation des groupes hydroxyle d'une cyclodextrine avec les fonctions carboxyliques de l'acide poly(carboxylique), préalablement séparées puis remélangées, et d'au moins un principe actif, dans laquelle l'acide poly(carboxylique) est l'acide citrique, la masse molaire moyenne en poids mesurée par chromatographie d'exclusion stérique de la fraction soluble dans l'eau du polymère de cyclodextrine est comprise entre 10 000 et 200 000 g.mol$^{-1}$, et le principe actif est un principe actif insoluble choisi parmi l'ibuprofène, la simvastatine, le piroxicam, l'itraconazole, le diclofénac de sodium, la mitomycine, l'oméprazole, l'ethoxzolamide, la chlorhexidine, la ciprofloxacine, le cisplatine et/ou le paclitaxel.

14. Composition compressible selon la revendication 13 constituée d'une fraction insoluble d'un polymère de cyclodextrine et d'au moins un principe actif.

15. Composition compressible selon la revendication 13 constituée d'une fraction soluble d'un polymère de cyclodextrine et d'au moins un principe actif.

16. Composition compressible selon la revendication 13 constituée d'un mélange de fractions insolubles et solubles de polymères de cyclodextrine et d'au moins un principe actif.

17. Composition compressible selon l'une quelconque des revendications 15 ou 16, dans laquelle la fraction soluble d'un polymère de cyclodextrine a été mélangée avec au moins un principe actif puis granulée par voie humide.

18. Composition compressible selon l'une quelconque des revendications 14 ou 16, dans laquelle la fraction insoluble d'un polymère de cyclodextrine a été mélangée avec au moins un principe actif.

19. Composition compressible selon l'une quelconque des revendications 13 à 18, sous forme de poudre de granulométrie comprise entre 250 et 1000 $\mu$m, de préférence entre 400 et 700 $\mu$m, de préférence encore inférieure à 630 $\mu$m.

20. Comprimé comprenant une composition compressible selon l'une quelconque des revendications 13 à 19.

21. Comprimé selon la revendication 20, dans lequel le comprimé a été préparé par compression directe.

22. Procédé de fabrication d'un comprimé comprenant les étapes consistant à :

   - préparer une composition compressible selon l'une quelconque des revendications 13 à 19 ;
   - comprimer ladite composition compressible afin de former un comprimé.

## Patentansprüche

1. Verwendung eines in Wasser unlöslichen Anteils eines durch Poly(carbonsäure) vernetzten Cyclodextrinpolymers, das durch Kondensation der Hydroxylgruppen eines Cyclodextrins mit den Carboxylfunktionen der Poly(carbonsäure) erhalten wird, eines in Wasser löslichen Anteils eines durch Poly(carbonsäure) vernetzten Cyclodextrinpolymers, das durch Kondensation der Hydroxylgruppen eines Cyclodextrins mit den Carboxylfunktionen der Poly(carbonsäure) erhalten wird, oder einer wasserunlöslichen und wasserlöslichen Mischung aus zuvor getrennten und anschließend erneut gemischten Anteilen, wobei durch Poly(carbonsäure) vernetzte Cyclodextrinpolymere durch Kondensation der Hydroxylgruppen eines Cyclodextrins mit den Carboxylfunktionen der Poly(carbonsäure) als einzigem Hilfsstoff in einer Tablette erhalten werden, wobei die Poly(carbonsäure) Zitronensäure ist, wobei die durch sterische Ausschlusschromatographie gemessene gewichtsmittlere Molmasse des in Wasser löslichen Anteils des Cyclodextrinpolymers zwischen 10.000 und 200.000 g.mol$^{-1}$ liegt.

2. Verwendung nach Anspruch 1, wobei der Hilfsstoff ein Komprimierungshilfsstoff, insbesondere zum direkten Komprimieren oder Komprimieren nach einem Nassgranulationsschritt, ein Solubilisierungshilfsstoff und/oder ein Komplexierungshilfsstoff für eine Tablette ist.

3. Verwendung nach Anspruch 1 oder 2, wobei ein in Wasser unlöslicher Anteil eines Cyclodextrinpolymers verwendet wird.

4. Verwendung nach Anspruch 1 oder 2, wobei ein in Wasser löslicher Anteil eines Cyclodextrinpolymers verwendet wird.

5. Verwendung nach Anspruch 4, wobei der in Wasser lösliche Anteil auf nassem Wege granuliert wird.

6. Verwendung nach Anspruch 1 oder 2, wobei eine Mischung aus in Wasser unlöslichen und in Wasser löslichen Anteilen von Cyclodextrinpolymeren verwendet wird.

7. Verwendung nach Anspruch 6, wobei das Verhältnis von dem in Wasser unlöslichen Anteil von Cyclodextrinpolymer/ in Wasser löslichen Anteil von Cyclodextrinpolymer zwischen 99:1 und 80:20 liegt.

8. Verwendung nach Anspruch 6, wobei das Verhältnis von unlöslichem Anteil von Cyclodextrinpolymer/löslichem Anteil von Cyclodextrinpolymer zwischen 80:20 und 25:75 liegt.

9. Verwendung einer Mischung aus in Wasser unlöslichen und in Wasser löslichen, zuvor getrennten und dann erneut gemischten Anteilen von Cyclodextrinpolymeren, die durch eine Poly(carbonsäure) vernetzt sind, wobei die Mischung durch Kondensation der Hydroxylgruppen eines Cyclodextrins mit Carboxylfunktionen einer Poly(carbonsäure) als Hilfsstoff in einer Tablette erhalten wird, wobei die Poly(carbonsäure) Zitronensäure ist, wobei die durch sterische Ausschlusschromatographie gemessene gewichtsmittlere Molmasse des in Wasser löslichen Anteils des Cyclodextrinpolymers zwischen 10.000 und 200.000 g.mol$^{-1}$ liegt.

10. Verwendung nach Anspruch 9, wobei der Hilfsstoff ein Komprimierungshilfsstoff, insbesondere zum direkten Komprimieren oder Komprimieren nach einem Nassgranulationsschritt, ein Solubilisierungshilfsstoff und/oder ein Komplexierungshilfsstoff für eine Tablette ist.

11. Verwendung nach Anspruch 10, wobei das Verhältnis von dem in Wasser unlöslichen Anteil von Cyclodextrinpolymer/ in Wasser löslichen Anteil von Cyclodextrinpolymer zwischen 99:1 und 80:20 liegt.

12. Verwendung nach Anspruch 10, wobei das Verhältnis von unlöslichem Anteil von Cyclodextrinpolymer/löslichem Anteil von Cyclodextrinpolymer zwischen 80:20 und 25:75 liegt.

13. Komprimierbare Zusammensetzung, die aus einem unlöslichen Anteil eines durch Poly(carbonsäure) vernetzten Cyclodextrinpolymers, das durch Kondensation der Hydroxylgruppen eines Cyclodextrins mit den Carboxylfunktionen der Poly(carbonsäure) erhalten wird, einem löslichen Anteil eines durch Poly(carbonsäure) vernetzten Cyclodextrinpolymers, das durch Kondensation der Hydroxylgruppen eines Cyclodextrins mit den Carboxylfunktionen der Poly(carbonsäure) erhalten wird oder einer Mischung aus zuvor getrennten und dann erneut gemischten unlöslichen und löslichen Anteilen von durch Poly(carbonsäure) vernetzten Cyclodextrinpolymeren, die durch Kondensation der Hydroxylgruppen eines Cyclodextrins mit den Carboxylfunktionen der Poly(carbonsäure) erhalten wird, und mindestens einem Wirkstoff, wobei die Poly(carbonsäure) Zitronensäure ist, besteht, wobei die durch sterischen Chromatographieausschluss gemessene gewichtsmittlere Molmasse des in Wasser löslichen Anteils des Cyclodextrinpolymers zwischen 10.000 und 200.000 g.mol$^{-1}$ liegt, und der Wirkstoff ein unlöslicher Wirkstoff ist, der aus Ibuprofen, Simvastatin, Piroxicam, Itraconazol, Diclofenac-Natrium, Mitomycin, Omeprazol, Ethoxzolamid, Chlorhexidin, Ciprofloxacin, Cisplatin und/oder Paclitaxel ausgewählt ist.

14. Komprimierbare Zusammensetzung nach Anspruch 13, die aus einem unlöslichen Anteil eines Cyclodextrinpolymers und mindestens einem Wirkstoff besteht.

15. Komprimierbare Zusammensetzung nach Anspruch 13, die aus einem löslichen Anteil eines Cyclodextrinpolymers und mindestens einem Wirkstoff besteht.

16. Komprimierbare Zusammensetzung nach Anspruch 13, die aus einer Mischung aus unlöslichen und löslichen Anteilen von Cyclodextrinpolymeren und mindestens einem Wirkstoff besteht.

**17.** Komprimierbare Zusammensetzung nach einem der Ansprüche 15 oder 16, wobei der lösliche Anteil eines Cyclodextrinpolymers mit mindestens einem Wirkstoff gemischt und dann auf nassem Wege granuliert wird.

**18.** Komprimierbare Zusammensetzung nach einem der Ansprüche 14 oder 16, wobei der unlösliche Anteil eines Cyclodextrinpolymers mit mindestens einem Wirkstoff gemischt wurde.

**19.** Komprimierbare Zusammensetzung nach einem der Ansprüche 13 bis 18 in Form eines Pulvers mit einer Teilchengröße zwischen 250 und 1000 $\mu$m, vorzugsweise zwischen 400 und 700 $\mu$m, stärker bevorzugt unter 630 $\mu$m.

**20.** Tablette, umfassend eine komprimierbare Zusammensetzung nach einem der Ansprüche 13 bis 19.

**21.** Tablette nach Anspruch 20, wobei die Tablette durch direktes Komprimieren hergestellt wurde.

**22.** Verfahren zum Herstellen einer Tablette, umfassend die folgenden Schritte:

   - Herstellen einer komprimierbaren Zusammensetzung nach einem der Ansprüche 13 bis 19;
   - Komprimieren der komprimierbaren Zusammensetzung, um eine Tablette zu bilden.


**Claims**

**1.** A use of a water-insoluble fraction of a cyclodextrin polymer cross-linked with a polycarboxylic acid obtained by condensation of the hydroxyl groups of a cyclodextrin with the carboxylic functions of the polycarboxylic acid, of a water-soluble fraction of a cyclodextrin polymer cross-linked with a polycarboxylic acid obtained by condensation of the hydroxyl groups of a cyclodextrin with the carboxylic functions of the polycarboxylic acid or of a mixture of water-insoluble and water-soluble fractions, previously separated and then mixed again, of cyclodextrin polymers cross-linked with a polycarboxylic acid obtained by condensation of the hydroxyl groups of a cyclodextrin with the carboxylic functions of the polycarboxylic acid as a single excipient in a tablet, wherein the polycarboxylic acid is citric acid, the weight average molar mass measured by steric exclusion chromatography of the water-soluble fraction of the cyclodextrin polymer is between 10.000 and 200.000 g.mol$^{-1}$.

**2.** The use according to claim 1, wherein the excipient is a compression excipient, especially direct compression or compression after a wet granulation step, solubilisation excipient and/or complexing excipient for a tablet.

**3.** The use according to claim 1 or 2, wherein a water-insoluble fraction of a cyclodextrin polymer is used.

**4.** The use according to claim 1 or 2, wherein a water-soluble fraction of a cyclodextrin polymer is used.

**5.** The use according to claim 4, wherein the water-soluble fraction has been wet granulated.

**6.** The use according to claim 1 or 2, wherein a mixture of water-insoluble and water-soluble fractions of cyclodextrin polymers is used.

**7.** The use according to claim 6, wherein the water-insoluble fraction of cyclodextrin polymer / water-soluble fraction of cyclodextrin polymer ratio is between 99:1 and 80:20.

**8.** The use according to claim 6, wherein the insoluble fraction of cyclodextrin polymer / soluble fraction of cyclodextrin polymer ratio is between 80:20 and 25:75.

**9.** A use of a mixture of water-insoluble and water-soluble fractions, previously separated and then mixed again, of cyclodextrin polymers cross-linked with a polycarboxylic acid obtained by condensation of the hydroxyl groups of a cyclodextrin with the carboxylic functions of the polycarboxylic acid as an excipient in a tablet, wherein the polycarboxylic acid is citric acid, the weight average molar mass measured by steric exclusion chromatography of the water-soluble fraction of the cyclodextrin polymer is between 10.000 and 200.000 g.mol$^{-1}$.

**10.** The use according to claim 9, wherein the excipient is a compression excipient, especially direct compression or compression after a wet granulation step, solubilisation excipient and/or complexing excipient for a tablet.

**11.** The use according to claim 10, wherein the water-insoluble fraction of cyclodextrin polymer / water-soluble fraction of cyclodextrin polymer ratio is between 99:1 and 80:20.

**12.** The use according to claim 10, wherein the insoluble fraction of cyclodextrin polymer / soluble fraction of cyclodextrin polymer ratio is between 80:20 and 25:75.

**13.** A compressible composition consisting of an insoluble fraction of a cyclodextrin polymer cross-linked with a polycarboxylic acid obtained by condensation of the hydroxyl groups of a cyclodextrin with the carboxylic functions of the polycarboxylic acid, of a soluble fraction of a cyclodextrin polymer cross-linked with a polycarboxylic acid obtained by condensation of the hydroxyl groups of a cyclodextrin with the carboxylic functions of the polycarboxylic acid or of a mixture of insoluble and soluble fractions of cyclodextrin polymers cross-linked with a polycarboxylic acid obtained by condensation of the hydroxyl groups of a cyclodextrin with the carboxylic functions of the polycarboxylic acid, previously separated and then mixed again, and of at least one active principle, wherein the polycarboxylic acid is citric acid, the weight average molar mass measured by steric exclusion chromatography of the water-soluble fraction of the cyclodextrin polymer is between 10.000 and 200.000 $g.mol^{-1}$, and the active principle is an insoluble active principle selected from ibuprofen, simvastatin, piroxicam, itraconazole, sodium diclofenac, mitomycin, omeprazole, ethoxzolamide, chlorhexidine, ciprofloxacin, cisplatin and/or paclitaxel.

**14.** The compressible composition according to claim 13 consisting of an insoluble fraction of a cyclodextrin polymer and of at least one active principle.

**15.** The compressible composition according to claim 13 consisting of a soluble fraction of a cyclodextrin polymer and of at least one active principle.

**16.** The compressible composition according to claim 13 consisting of a mixture of insoluble and soluble fractions of cyclodextrin polymers and of at least one active principle.

**17.** The compressible composition according to any of claims 15 or 16, wherein the soluble fraction of a cyclodextrin polymer has been mixed with at least one active principle and then wet granulated.

**18.** The compressible composition according to any of claims 14 or 16, wherein the insoluble fraction of a cyclodextrin polymer has been mixed with at least one active principle.

**19.** The compressible composition according to any of claims 13 to 18, as a powder with a grain size between 250 and 1000 $\mu$m, preferably between 400 and 700 $\mu$m, still preferably less than 630 $\mu$m.

**20.** A tablet comprising a compressible composition according to any of claims 13 to 19.

**21.** The tablet according to claim 20, wherein the tablet has been prepared by direct compression.

**22.** A method for manufacturing a tablet comprising the steps of:

- preparing a compressible composition according to any of claims 13 to 19;
- compressing said compressible composition in order to form a tablet.

**Figure 1**

**Figure 2**

**Figure 3**

**Figure 4**

Figure 5

a)

b)

c)

**Figure 6**

**Figure 7**

**Figure 8**

(1) (2) (3) (4)

**Figure 9**

**Figure 10**

**Figure 11**

$(poly-\beta CD)_{1-x}$ $(mannitol)_X$    $Tg=f(X)$

Figure 12

Figure 13

**Figure 14**

**Figure 15**

**Figure 16**

**Figure 17**

**Figure 18**

**Figure 19**

**Figure 20**

**Figure 21**

## RÉFÉRENCES CITÉES DANS LA DESCRIPTION

### Documents brevets cités dans la description

- CN 1879887 A
- IN 2010MU03419 A

- WO 0047630 A

### Littérature non-brevet citée dans la description

- **B. MARTEL ; D. RUFFIN ; M. MORCELLET ; M. WELTROWSKI ; Y. LEKCHIRI.** Water soluble polymers and gels from polycondensation between cyclodextrins and polycarboxylic acids : study of the preparation parameters. *J. Appl. Polym. Sci.,* 2005, vol. 97, 433-442

- **MARTEL et al.** *Green Chem,* 2015, vol. 17, 2444-2454
- Comprehensive supramolecular chemistry. Cyclodextrins. Pergamon, 1996, vol. 3